# DEMANDE DE BREVET EUROPEEN

(11) **EP 1 561 753 A2**
(43) Date de publication de la demande: **10.08.2005**
(21) Numéro de dépôt: 05290220.2
(22) Date de dépôt: 01.02.2005
(51) Int. Cl.: C07D 491/14, C07D 491/22, A61K 31/4375, A61K 31/4985

(54) **Dérivés de benzo[b]chroménonapthyridin-7-one et de pyrano[2',3':7,8]quino[2,3-b]quinoxalin-7-one, leur procédé de préparation, les compositions pharmaceutiques qui les contiennent et leurs propriétés antitumorales pour le traitement du cancer**

(30) Priorité: 03.02.2004 FR 0400988
(71) Demandeur: Les Laboratoires Servier, 92415 Courbevoie Cedex (FR)
(72) Inventeur: Koch, Michel, 78170 La Celle Saint Cloud (FR); Tillequin, Francois, 75014 Paris (FR); Michel, Sylvie, 75002 Paris (FR); Hickman, John, 75017 Paris (FR); Pierre, Alain, 78580 les Alluets Le Roi (FR); Leonce, Stéphane, 78000 Versailles (FR); Pfeiffer, Bruno, 95320 Saint Leu La Foret (FR); Renard, Pierre, 78150 Le Chesnay (FR)

(57) **Abrégé**

Composés de formule (I): 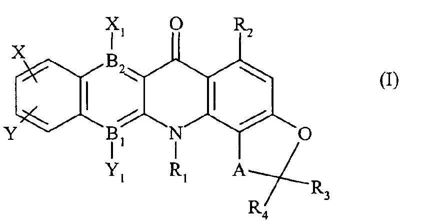 dans laquelle:
- B₁, B₂ représentent un atome de carbone ou d'azote,
- X, Y, X₁ et Y₁ représentent un groupement choisi parmi hydrogène, halogène, hydroxy, alkoxy, nitro, cyano, trihalogénoalkyle et NRₐR_{b} dans lequel Rₐ et R_{b} sont tels que définis dans la description,
- R₁ représente un atome d'hydrogène ou un groupement alkyle,
- R₂ représente un groupement choisi parmi atome d'hydrogène, alkyle, -OR''ₐ,-NR'ₐR'_{b}, -O-Tₐ-OR''ₐ, -NR''ₐ-Tₐ-NR'ₐR'_{b}, NR''ₐ-C(O)-TₐH, -O-C(O)-TₐH, -O-Tₐ-NRₐ'R'_{b}, -NR''ₐ-Tₐ-OR''ₐ, - NR''ₐ-Tₐ-CO₂R''ₐ, -NR''ₐ-C(O)-Tₐ-NR'ₐR'_{b} dans lesquels R'ₐ, R'_{b}, R''ₐ et Tₐ sont tels que définis dans la description,
- R₃, R₄ représentent un atome d'hydrogène ou un groupement alkyle,
- A représente un groupement de formule -CH(R₅)-CH(R₆)-, -CH=C(R₇)-, -C(R₇)=CH-, -C(O)-CH(R₈)-, -CH(R₈)-C(O)- dans lesquelles R₅, R₆, R₇ et R₈ sont tels que définis dans la description,
leurs isomères définis dans la description, N-oxydes, ainsi que leurs sels d'addition à un acide ou à une base pharmaceutiquement acceptable, avec des propriétés antitumorales les rendant utiles dans le traitement du cancer, médicaments les contenant.

## Description

La présente invention concerne de nouveaux dérivés de benzo[b]chroméno-naphthyridin-7-one et de pyrano[2',3':7,8]quino[2,3-*b*]quinoxalin-7-one, leur procédé de préparation et les compositions pharmaceutiques qui les contiennent.

Les composés de l'invention constituent des dérivés de l'acronycine qui est un alcaloïde présentant des propriétés antitumorales mises en évidence dans des modèles expérimentaux (*J. Pharm.. Sci.,* 1966, 55 (8), 758-768). Cependant, malgré un spectre d'activité assez large, l'acronycine est peu puissante et modérément active. De plus, ce produit présente une faible solubilité limitant sa biodisponibilité ainsi que son utilisation dans des compositions pharmaceutiques administrables par voie intraveineuse.

Diverses modifications ont été réalisées sur cette molécule, comme celles décrites dans *J. Med. Chem.,* 1996, 39, 4762-4766, EP 1 042 326, EP 1 061 081 ou EP 1 297 835, et qui ont permis d'améliorer significativement la puissance, l'efficacité antitumorale et la solubilité de ces produits. Néanmoins, les besoins de la thérapeutique anticancéreuse exigent le développement constant de nouveaux agents antitumoraux, dans le but d'obtenir des médicaments à la fois plus actifs et mieux tolérés. Plus particulièrement, les tumeurs solides posent un problème majeur à la chimiothérapie anticancéreuse, de par leur résistance intrinsèque et/ou acquise, aux produits existants. Il est donc primordial d'avoir accès à une gamme la plus large possible de produits exprimant une activité cytotoxique forte afin de pouvoir disposer de traitements des plus efficaces sur l'ensemble des affections tumorales.

Les composés de l'invention, outre le fait qu'ils soient nouveaux, présentent une activité cytotoxique *in vitro* et *in vivo* surprenante et supérieure à celle observée jusqu'ici. Ainsi, les composés découverts par la Demanderesse possèdent des propriétés antitumorales qui les rendent particulièrement utiles pour le traitement des cancers. Parmi les types de cancers qui peuvent être traités par les composés de la présente invention, on peut citer à titre non limitatif les adénocarcinomes et carcinomes, sarcomes, gliomes et leucémies.

Plus particulièrement, la présente invention concerne les composés de formule (I) : 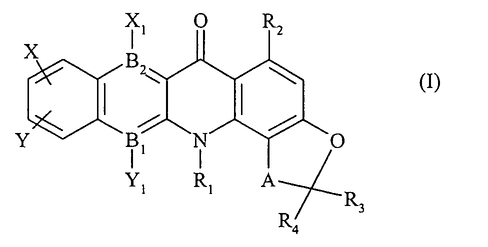 dans laquelle :
- B₁ et B₂, identiques ou différents, indépendamment l'un de l'autre, représentent chacun un atome de carbone ou un atome d'azote étant entendu qu'au moins un des deux groupements B₁ ou B₂ représente un atome d'azote,
- X et Y, identiques ou différents, indépendamment l'un de l'autre, représentent chacun un ou plusieurs groupements choisis parmi:
   - atome d'hydrogène, halogène,
   - groupement hydroxy, alkoxy (C₁-C₆) linéaire ou ramifié, nitro, cyano, alkyle (C₁-C₆) linéaire ou ramifié, alkényle (C₂-C₆) linéaire ou ramifié, polyhalogénoalkyle (C₁-C₆) linéaire ou ramifié, ou
   - groupement de formule -NRₐR_{b} dans lequel:
      Rₐ et R_{b}, identiques ou différents, indépendamment l'un de l'autre, représentent chacun un groupement choisi parmi atome d'hydrogène, -C(O)-CF₃, -C(O)-NH₂ ou groupement alkyle (C₁-C₆) linéaire ou ramifié, éventuellement substitué par un groupement NR'ₐR'_{b} dans lequel :
         R'ₐ et R'_{b}, identiques ou différents, indépendamment l'un de l'autre, représentent chacun un groupement choisi parmi atome d'hydrogène, groupement alkyle (C₁-C₆) linéaire ou ramifié, ou R'ₐ et R'_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
   ou Rₐ et R_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
   - X₁ et Y₁, identiques ou différents, indépendamment l'un de l'autre, représentent chacun un groupement choisi parmi les substituants tels que définis pour X et Y,
   étant entendu que lorsque B₁ et/ou B₂ représentent un atome d'azote, B₁ et B₂ ne portent pas de substituants Y₁ et X₁ respectivement,
   - R₁ représente un atome d'hydrogène ou un groupement alkyle (C₁-C₆) linéaire ou ramifié,
   - R₂ représente un groupement choisi parmi atome d'hydrogène, groupement alkyle (C₁-C₆) linéaire ou ramifié, -OR"ₐ ; -NR'ₐR'_{b} ; O-Tₐ-OR"ₐ ; -NR"ₐ-Tₐ-NR'ₐR'_{b}; -NR"ₐ-C(O)-TₐH ; O-C(O)-TₐH ; -O-Tₐ-NR'ₐR'_{b} ; -NR"ₐ-Tₐ-OR"ₐ; -NR"ₐ-Tₐ-CO₂R"ₐ ; -NR"ₐ-C(O)-Tₐ-NR'ₐR'_{b},
      dans lesquels :
      * Tₐ représente une chaîne alkylène (C₁-C₆) linéaire ou ramifiée,
      * R'ₐ et R'_{b} sont tels que définis précédemment,
      * R"ₐ représente un groupement choisi parmi atome d'hydrogène, groupement alkyle (C₁-C₆) linéaire ou ramifié,
   - R₃ et R₄, identiques ou différents, indépendamment l'un de l'autre, représentent chacun un atome d'hydrogène ou un groupement alkyle (C₁-C₆) linéaire ou ramifié, ou R₃ et R₄ forment ensemble avec l'atome de carbone qui les portent, un cycle de 3 à 6 chaînons, monocyclique,
   - A représente un groupement de formule :
      a) -CH(R₅)-CH(R₆)- dans lequel :
         * *R*_{*5*} *et R*_{*6*}*, identiques ou différents, indépendamment l'un de l'autre représentent chacun un groupement choisi parmi :*
            1) atome d'hydrogène,
            2) groupement OR_{c}, NR_{c}R_{d}, SR_{c}, dans lesquels :
               - R_{c}, R_{d}, identiques ou différents, indépendamment l'un de l'autre, représentent chacun un groupement choisi parmi atome d'hydrogène, groupement alkyle (C₁-C₆) linéaire ou ramifié, ou un groupement aryle, arylalkyle (C₁-C₆) linéaire
               ou ramifié,
               ou un groupement C(O)-Rₑ dans lequel Rₑ représente un groupement choisi parmi atome d'hydrogène, groupement aryle, ou NR"'ₐR"'_{b} dans lequel R"'ₐ et R"'_{b}, identiques, représentent un atome d'hydrogène ou R"'ₐ et R"'_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
            3) W₁-C(W₂)-U-V dans lequel :
               α) W₁ représente un atome d'oxygène, de soufre ou NR_{c} (dans lequel R_{c} est tel que défini précédemment),
               β) W₂ représente un atome d'oxygène ou un atome de soufre,
               γ) U représente une chaîne alkylène (C₁-C₈) linéaire ou ramifiée ou une chaîne alkénylène (C₂-C₈) linéaire ou ramifiée,
               δ) V représente un groupement choisi parmi :
                  - atome d'hydrogène,
                  - groupement aryle,
                  - groupements OR_{c}, CO₂R_{c}, COR_{c}, CONR'ₐR'_{b}, NR_{c}R_{d}, N(R_{c})-CO₂R'_{c}, N(R_{c})-COR'_{c}, dans lesquels R'ₐ, R'_{b}, R_{c} et R_{d} sont tels que définis précédemment et R'_{c} représente un groupement choisi parmi atome d'hydrogène, groupement alkyle (C₁-C₆) linéaire ou ramifié, ou un groupement aryle, arylalkyle (C₁-C₆) linéaire ou ramifié,
               ε) U représente une liaison lorsque W₂ ne représente pas un atome d'oxygène et lorsque V ne représente pas un groupement choisi parmi:
                  - atome d'hydrogène,
                  - groupement aryle,
                  - NH₂,
            4) W₁-C(W₂)-W₃-T₁ dans lequel :
               α) W₁et W₂ sont tels que définis précédemment,
               β) W₃ représente un atome d'oxygène, de soufre ou NR_{c} dans lequel R_{c} est tel que défini précédemment,
               γ) T₁ représente un groupement choisi parmi :
                  - atome d'hydrogène,
                  - alkyle (C₁-C₆) linéaire ou ramifié,
                  - alkényle (C₂-C₆) linéaire ou ramifié,
                  - aryle, arylalkyle (C₁-C₆) linéaire ou ramifié,
                  - chaîne alkylène (C₁-C₆) linéaire ou ramifiée ou une chaîne alkénylène (C₂-C₆) linéaire ou ramifiée, chacune étant substituée par un groupement OR_{c}
               dans lequel R_{c} est tel que défini précédemment ou NR'ₐR'_{b} dans lequel R'ₐ et R'_{b} sont tels que définis précédemment,
            5) W₁-S(O)ₙ W₃-T₁ dans lequel :
               α) W₁, W₃ et T₁ sont tels que définis précédemment,
               β) n représente un nombre entier choisi parmi 1 et 2,
            6) W₁-S(O)ₙ-T₁ dans lequel W₁, T₁ et n sont tels que définis précédemment,
            7) C(W₂)-T₁ dans lequel W₂ et T₁ sont tels que définis précédemment,
      ** ou R*_{*5*} *et R*_{*6*}*, forment ensemble :*
      1) un groupement 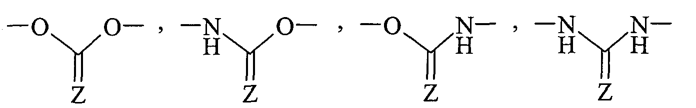 dans lesquels Z représente un atome d'oxygène ou un atome de soufre,
      2) un groupement -O-(CH₂)ₘ-O- dans lequel m représente un nombre entier compris entre 1 et 4 inclus,
      3) un groupement 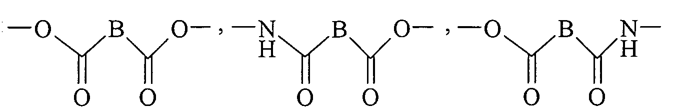 dans lesquels B représente une liaison simple, une chaîne alkylène (C₁-C₆) linéaire ou ramifiée ou une chaîne alkénylène (C₂-C₆) linéaire ou ramifiée,
         ** ou R*_{*5*} *et R*_{*6*}*, forment ensemble avec les atomes de carbone qui les portent, un groupement oxirane ou un groupement aziridine,* éventuellement substitué sur l'atome d'azote par un groupement alkyle (C₁-C₆) linéaire ou ramifié,
      b) -CH=C(R₇)- ou -C(R₇)=CH- dans lesquels R₇ représente un groupement choisi parmi :
         - atome d'hydrogène,
         - groupement OR"ₐ, W₁-C(W₂)-U-V, W₁-C(W₂)-W₃-T₁, W₁-S(O)ₙ-W₃-T₁, W₁-S(O)ₙ-T₁ ou C(W₂)-T₁ dans lesquels R"ₐ, W₁, W₂, U, V, W₃, T₁ et n sont tels que définis précédemment,
      c) -C(O)-CH(R₈)- ou - CH(R₈)-C(O)- dans lesquels R₈ représente un groupement choisi parmi :
         - atome d'hydrogène,
         - alkylcarbonyloxy (C₁-C₆) linéaire ou ramifié, groupement OR"ₐ dans lequel R"ₐ est tel que défini précédemment,
leurs énantiomères, diastéréoisomères, N-oxydes, ainsi que leurs sels d'addition à un acide ou à une base pharmaceutiquement acceptable,
étant entendu que par aryle, on comprend un groupement phényle ou naphtyle, comportant éventuellement une ou plusieurs substitutions, identiques ou différentes, choisies parmi alkyle (C₁-C₆) linéaire ou ramifié, alkényle (C₂-C₆) linéaire ou ramifié, hydroxy, halogène, carboxy, nitro, amino, alkylamino ou dialkylamino (C₁-C₆) linéaire ou ramifié, alkoxy (C₁-C₆) linéaire ou ramifié, acyle (C₁-C₆) linéaire ou ramifié, et alkylcarbonyloxy (C₁-C₆) linéaire ou ramifié,
parmi les hétérocycles de 5 à 7 chaînons, monocyclique, contenant éventuellement au sein du système cyclique un second hétéroatome choisi parmi oxygène et azote, on peut citer à titre non limitatif les groupements pyrrolidinyle, isoxazolidinyle, oxazolidinyle, pyrazolidinyle, imidazolidinyle, pipéridinyle, morpholinyle, hexahydropyridiazinyle, hexahydropyrimidinyle, pipérazinyle, azépanyle, oxazépanyle, diazépanyle,
parmi les cycles de 3 à 6 chaînons, monocyclique, on peut citer à titre non limitatif les groupements cyclopropyle, cyclobutyle, cyclopentyle et cyclohexyle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, la lysine, etc...

Parmi les bases pharmaceutiquement acceptables, on peut citer à titre non limitatif l'hydroxyde de sodium, l'hydroxyde de potassium, la triéthylamine, la tertbutylamine, etc...

Selon une variante avantageuse de l'invention, les composés préférés sont les composés de formule (IA) : 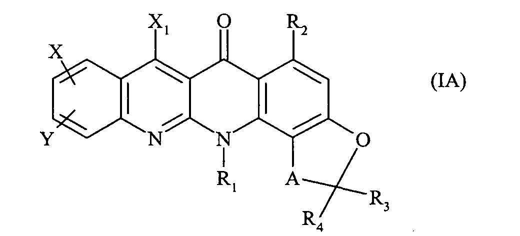 dans laquelle X, Y, X₁, R₁, R₂, R₃, R₄ et A sont tels que définis dans la formule (I).

Selon une deuxième variante avantageuse de l'invention, les composés préférés sont les composés de formule (IB) : 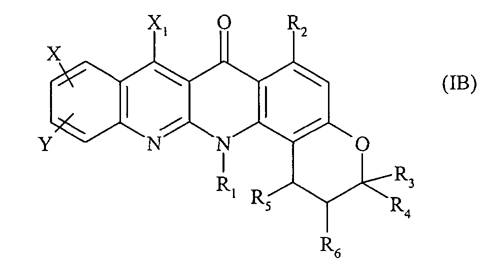 dans laquelle X, Y, X₁, R₁, R₂, R₃, R₄, R₅ et R₆ sont tels que définis dans la formule (I).

Selon une troisième variante avantageuse de l'invention, les composés préférés sont les composés de formule (IC) : 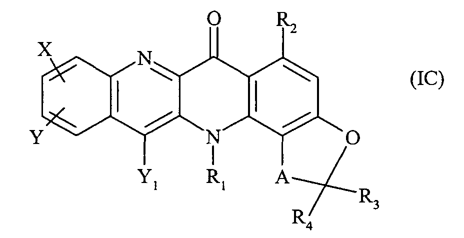 dans laquelle X, Y, Y₁, R₁, R₂, R₃ R₄ et A sont tels que définis dans la formule (I).

Selon une quatrième variante avantageuse de l'invention, les composés préférés sont les composés de formule (ID) : 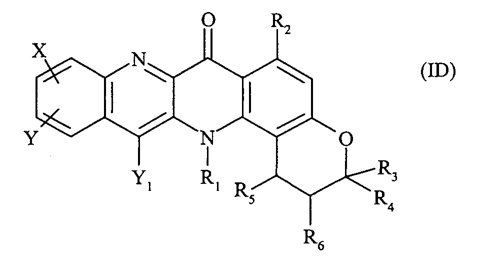 dans laquelle X, Y, Y₁, R₁, R₂, R₃, R₄, R₅ et R₆ sont tels que définis dans la formule (I).

Selon une cinquième variante avantageuse de l'invention, les composés préférés sont les composés de formule (IE) : 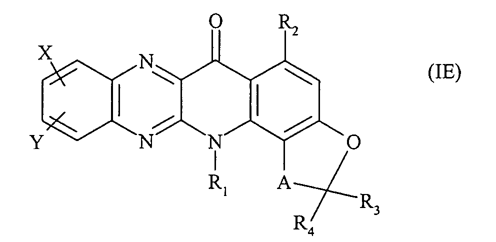 dans laquelle X, Y, R₁, R₂, R₃, R₄ et A sont tels que définis dans la formule (I).

Selon une sixième variante avantageuse de l'invention, les composés préférés sont les composés de formule (IF) : 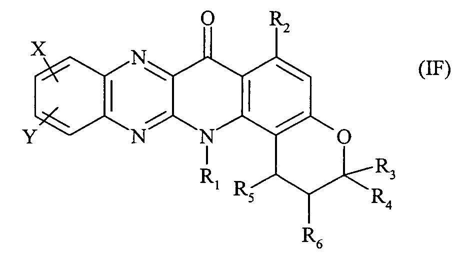 dans laquelle X, Y, R₁, R₂, R₃, R₄, R₅ et R₆ sont tels que définis dans la formule (I).

Les composés préférés de formules (IB), (ID) et (IF) sont les composés dans lesquels R₅ et R₆, identiques, représentent un groupement de formule -OR_{c}, W₁-C(W₂)-U-V ou R₅ et R₆ forment ensemble un groupement 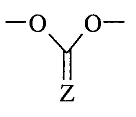 dans lesquels R_{c}, W₁, W₂, U, V et Z sont tels que définis dans la formule (I).

D'une façon particulièrement intéressante, les composés préférés de formules (IB), (ID) et (IF) sont les composés dans lesquels R₅ et R₆, identiques, représentent un groupement de formule -OR_{c} dans laquelle R_{c} représente un atome d'hydrogène ou un groupement alkyle (C₁-C₆) linéaire ou ramifié.

D'une autre façon particulièrement intéressante, les composés préférés de formules (IB), (ID) et (IF) sont les composés dans lesquels R₅ et R₆, identiques, représentent un groupement de formule W₁-C(W₂)-U-V dans laquelle W₁ et W₂ représentent un atome d'oxygène, U représente une chaîne alkylène (C₁-C₆) linéaire ou ramifié et V représente un atome d'hydrogène.

D'une autre façon très intéressante, les composés préférés de formules (IB), (ID) et (IF) sont les composés dans lesquels R₅ et R₆ forment ensemble un groupement 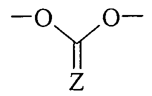 dans lequel Z représente un atome d'oxygène. Selon une septième variante avantageuse de l'invention, les composés préférés sont les composés de formule (IG) : 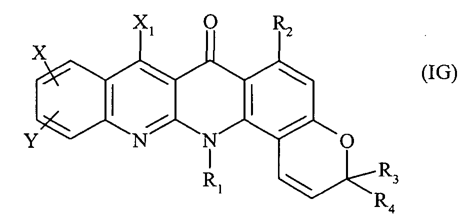 dans laquelle X, Y, X₁, R₁, R₂, R₃ et R₄ sont tels que définis dans la formule (I).

Selon une huitième variante avantageuse de l'invention, les composés préférés sont les composés de formule (IH) : 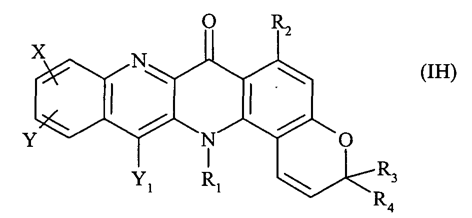 dans laquelle X, Y, Y₁, R₁, R₂, R₃ et R₄ sont tels que définis dans la formule (I).

Selon une neuvième variante avantageuse de l'invention, les composés préférés sont les composés de formule (IJ) : 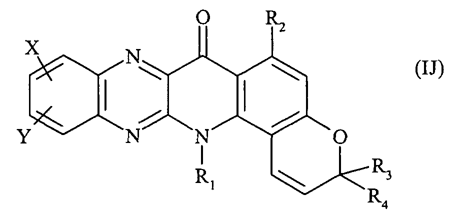 dans laquelle X, Y, R₁, R₂, R₃ et R₄ sont tels que définis dans la formule (I).

Les substituants R₃ et R₄ préférés selon l'invention sont le groupement alkyle (C₁-C₆) linéaire ou ramifié.

Le substituant R₂ préféré selon l'invention est le groupement -OR"ₐ dans lequel R"ₐ est tel que défini dans la formule (I).
Encore plus préférentiellement, le substituant R₂ préféré selon l'invention est le groupement -OR"ₐ dans lequel R"ₐ représente un groupement alkyle (C₁-C₆) linéaire ou ramifié, et encore plus préférentiellement un groupement méthyl.

Les substituants X, Y, X₁ et Y₁ préférés selon l'invention sont l'atome d'hydrogène.

D'une façon particulièrement avantageuse, les composés préférés de l'invention sont le:
- (±)-*cis*-1,2-dihydroxy-6-méthoxy-3,3,14-triméthyl-1,2,3,14-tétrahydro-7*H*-benzo[*b*] chroméno[6,5-*g*] [1,8]naphthyridin-7-one,
- (±)-*cis*-1,2-diacétoxy-6-méthoxy-3,3,14-triméthyl-1,2,3,14-tétrahydro-7*H*-benzo[*b*] chroméno[6,5-*g*] [1,8]naphthyridin-7-one,
- (±)-*cis*-7-méthoxy-4,4,15-triméthyl-15,15c-dihydro-4*H*-benzo[*b*][1,3]dioxolo [4',5':3,4]chroméno[6,5-g] [1,8]naphthyridine-2,8(3a*H*)-dione,

Les énantiomères, diastéréoisomères, N-oxydes, ainsi que les sels d'addition à un acide ou à une base, pharmaceutiquement acceptable, des composés préférés font partie intégrante de l'invention.

La présente invention s'étend également au procédé de préparation des composés de formule (I), caractérisé en ce qu'on utilise comme produit de départ un composé de formule (II) : 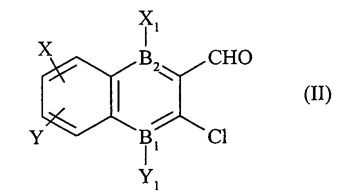 dans laquelle X, Y, X₁, Y₁, B₁ et B₂ sont tels que définis dans la formule (I), composés de formule (II) qui sont mis en présence de pyridinium dichromate pour conduire aux composés de formule (III) : 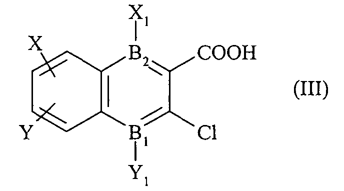 dans laquelle X, Y, X₁, Y₁, B₁ et B₂ sont tels que définis dans la formule (I),
composés de formule (III) qui sont traités en milieu basique et anhydre par un composé de formule (IV) : 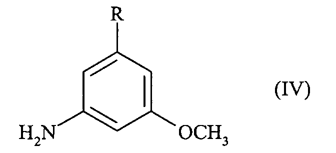 dans laquelle R représente un atome d'hydrogène, un groupement hydroxy, un groupement alkyle (C₁-C₆) linéaire ou ramifié ou un groupement alkoxy (C₁-C₆) linéaire ou ramifié pour conduire aux composés de formule (V) : 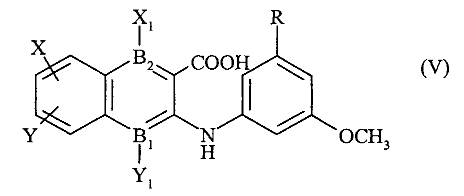 dans laquelle X, Y, X₁, Y₁, B₁, B₂ et R sont tels que définis dans la formule (I),
composés de formule (V) qui sont mis en présence d'acide polyphosphorique pour conduire aux composés de formule (VI) : 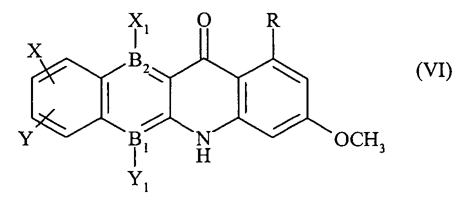 dans laquelle X, Y, X₁, Y₁, B₁, B₂ et R sont tels que définis précédemment,

### composés de formule (VI) :

**a)** dont l'atome d'azote est substitué, par action d'un halogénure d'alkyle ou d'un sulfate de dialkyle en présence d'un agent de déprotonation dans un solvant polaire aprotique pour conduire aux composés de formule (VII) : 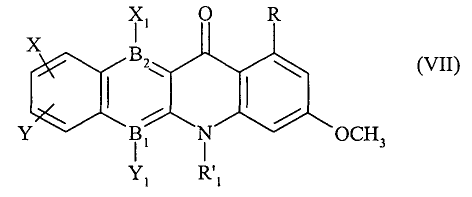 dans laquelle R'₁ représente un groupement alkyle (C₁-C₆) linéaire ou ramifié et X, Y, X₁, Y₁, B₁, B₂ et R sont tels que définis précédemment,
   composés de formule (VII) qui sont traités par une solution d'acide bromhydrique dans de l'acide acétique, pour conduire aux composés de formule (VIII) : 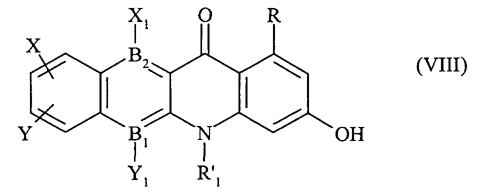 dans laquelle X, Y, X₁, Y₁, B₁, B₂, R et R'₁ sont tels que définis précédemment,
   composés de formule (VIII) qui sont ensuite traités en condition basique dans un solvant aprotique anhydre par un alcyne de formule (IX) : 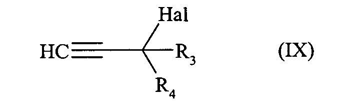 dans laquelle Hal représente un atome d'halogène et R₃ et R₄ sont tels que définis dans la formule (I), pour conduire aux composés de formule (X) : 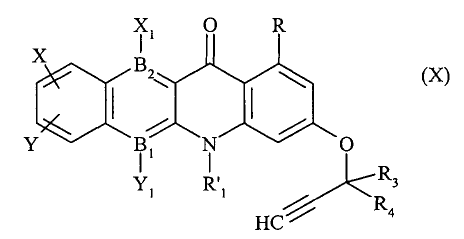 dans laquelle X, Y, X₁, Y₁, B₁, B₂, R, R'₁, R₃ et R₄ sont tels que définis précédemment, composés de formule (X) qui sont mis sous reflux dans du diméthylformamide anhydre pour conduire aux composés de formule (I/a), cas particulier des composés de formule (I) : 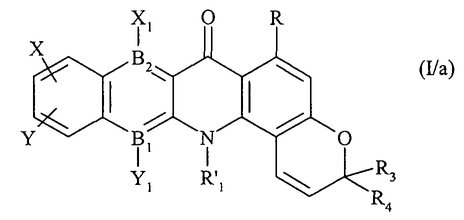 dans laquelle X, Y, X₁, Y₁, B₁, B₂, R, R'₁, R₃ et R₄ sont tels que définis précédemment,
**b)** soit soumis aux mêmes conditions que les composés de formule (VII) pour conduire aux composés de formule (XI) : 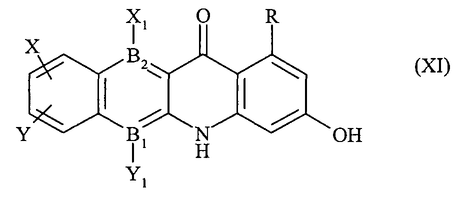 dans laquelle X, Y, X₁, Y₁, B₁, B₂ et R sont tels que définis précédemment,
   composés de formule (XI) qui sont soumis successivement aux mêmes conditions que les composés de formule (VIII) et (X) pour conduire aux composés de formule (I/b), cas particulier des composés de formule (I) : 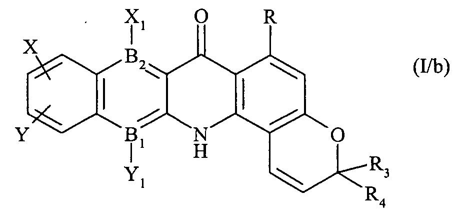 dans laquelle X, Y, X₁, Y₁, B₁, B₂, R, R₃ et R₄ sont tels que définis précédemment,
l'ensemble des composés (I/a) et (I/b) formant les composés de formule (I/c), cas particulier des composés de formule (I) : 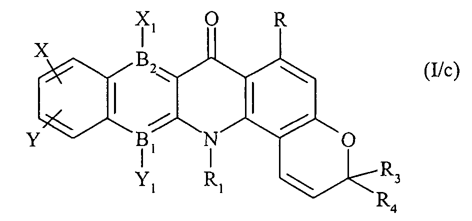 dans laquelle R₁ est tel que défini dans la formule (I) et X, Y, X₁, Y₁, B₁, B₂, R, R₃ et R₄ sont tels que définis précédemment,
composés de formule (I/c) qui sont éventuellement soumis à l'action d'un agent alkylant ou d'un agent acylant pour conduire aux composés de formule (I/d), cas particulier des composés de formule (I) : 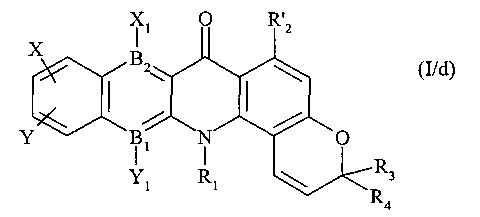 dans laquelle X, Y, X₁, Y₁, B₁, B₂, R₁, R₃ et R₄ sont tels que définis précédemment, et R'₂ représente un groupement choisi parmi -OR"ₐ₁, O-Tₐ-OR"ₐ, -O-C(O)-TₐH, -O-Tₐ-NR'ₐR'_{b} dans lesquels R'ₐ, R'_{b}, R"ₐ et Tₐ sont tels que définis dans la formule (I) et R"ₐ₁ représente un groupement alkyle (C₁-C₆) linéaire ou ramifié,
composés de formule (I/d) qui sont éventuellement traités, dans le cas où R'₂ représente un groupement
-OR"ₐ₁ dans lequel R"ₐ₁ est tel que défini précédemment par un composé de formule (XII) :

HNR₁₀R₁₁ (XII

dans laquelle R₁₀ représente un groupement choisi parmi R'ₐ et R"ₐ et R₁₁ représente un groupement choisi parmi R'_{b}, -Tₐ-NR'ₐR'_{b}, -C(O)-TₐH, -Tₐ-OR"ₐ, -Tₐ-CO₂R"ₐ et -C(O)-Tₐ-NR'ₐR'_{b}, dans lesquels R'ₐ, R'_{b}, R"ₐ et Tₐ sont tels que définis précédemment, pour conduire aux composés de formule (I/e), cas particulier des composés de formule (I) : 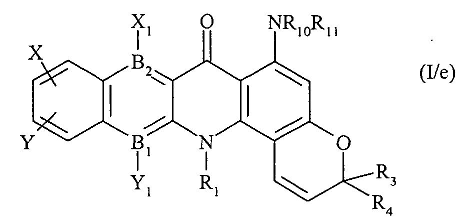 dans laquelle X, Y, X₁, Y₁, B₁, B₂, R₁, R₃ R₄, R₁₀ et R₁₁ sont tels que définis précédemment,
l'ensemble des composés de formule (I/c), (I/d) et (I/e) formant les composés de formule (I/f), cas particulier des composés de formule (I) : 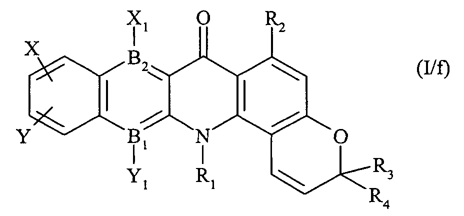 dans laquelle X, Y, X₁, Y₁, B₁, B₂, R₁, R₂, R₃ et R₄ sont tels que définis dans la formule (I),

### composés de formule (I/f) pouvant être soumis,

**a)** soit à l'action d'un agent réducteur, pour conduire aux composés de formule (I/g), cas particulier des composés de formule (I) : 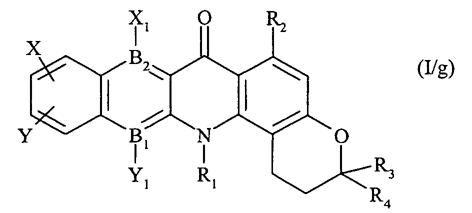 dans laquelle X, Y, X₁, Y₁, B₁, B₂, R₁, R₂, R₃ et R₄ sont tels que définis précédemment,
**b)** soit à l'action du tétroxyde d'osmium en milieu polaire et en présence de 4-méthylmorpholine-N-oxyde, pour conduire aux composés de formules (I/h₁) et (I/h₂), cas particulier des composés de formule (I) : 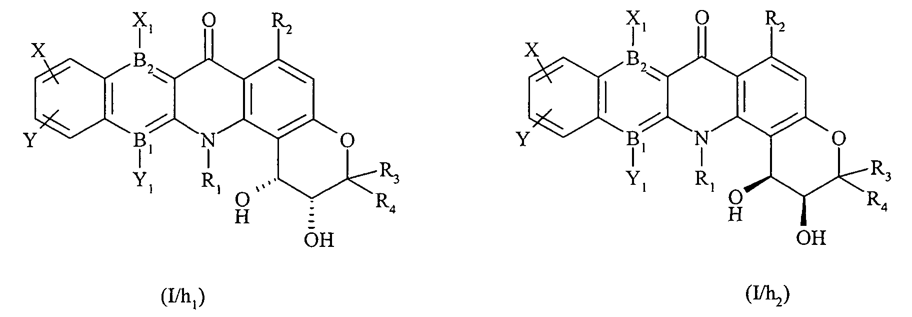 dans laquelle X, Y, X₁, Y₁, B₁, B₂, R₁, R₂, R₃ et R₄ sont tels que définis précédemment,
   l'ensemble des composés de formule (I/h₁) et (I/h₂) formant les composés *cis*-diol de formule (*cis*-I/h), cas particulier des composés de formule (I) : 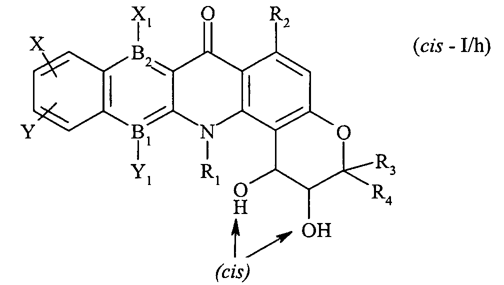 dans laquelle X, Y, X₁, Y₁, B₁, B₂, R₁, R₂, R₃ et R₄ sont tels que définis précédemment,
   composés cis-diol de formule (*cis-*I/h) qui sont soumis éventuellement à l'action d'un composé de formule (XIII) : 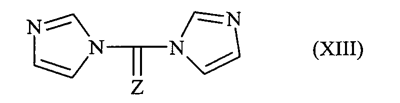 dans laquelle Z est tel que défini dans la formule (I) pour conduire aux composés de formule (*cis-*I/i), cas particulier des composés de formule (I) : 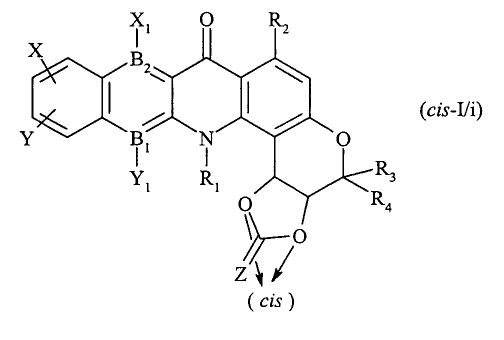 dans laquelle X, Y, X₁, Y₁, B₁, B₂, R₁, R₂, R₃ et R₄ sont tels que définis précédemment,
c) soit à l'action du permanganate de potassium en milieu polaire, pour conduire aux composés de formule (I/j), cas particulier des composés de formule (I) : 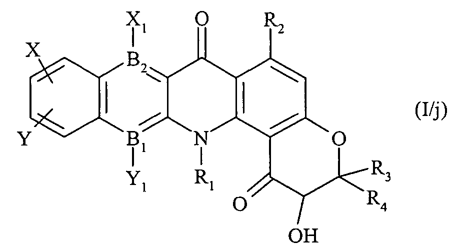
dans laquelle X, Y, X₁, Y₁, B₁, B₂, R₁, R₂, R₃ et R₄ sont tels que définis précédemment,

### composés de formule (I/j) pouvant être soumis :

α) soit à l'action d'un agent alkylant ou d'un agent acylant, pour conduire aux composés de formule (I/k), cas particulier des composés de formule (I) : 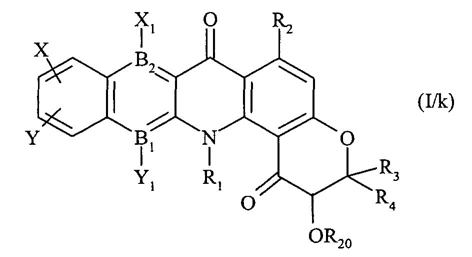 dans laquelle X, Y, X₁, Y₁, B₁, B₂, R₁, R₂, R₃ et R₄ sont tels que définis précédemment, et R₂₀ représente un groupement choisi parmi alkyle (C₁-C₆) linéaire ou ramifié ou alkylcarbonyl (C₁-C₆) linéaire ou ramifié,
β) soit à l'action d'un groupement protecteur P_{G} pour la fonction hydroxy puis à l'action de P₂S₅, pour conduire aux composés de formule (XIV) : 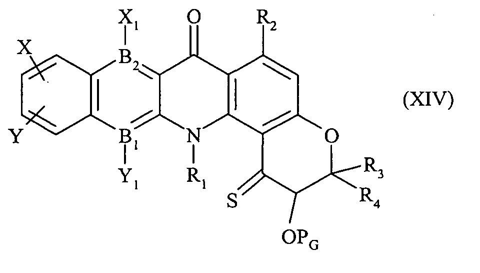 dans laquelle X, Y, X₁, Y₁, B₁, B₂, R₁, R₂, R₃, R₄ et P_{G} sont tels que définis précédemment,
   composés de formule (XIV) qui sont traités par un agent de réduction, puis soumis à une réaction de déprotection de la fonction hydroxy, pour conduire aux composés de formule (I/1), cas particulier des composés de formule (I) : 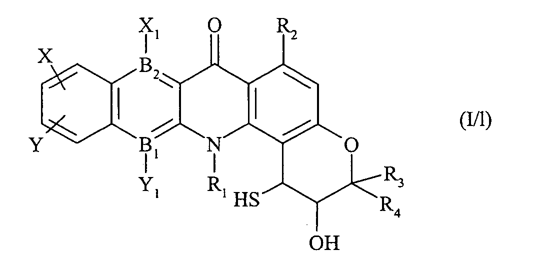 dans laquelle X, Y, X₁, Y₁, B₁, B₂, R₁, R₂, R₃ et R₄ sont tels que définis précédemment,
   composés de formule (I/1) qui sont éventuellement soumis à l'action d'un composé de formule (XV) :

   Hal - G₁ (XV)

   dans laquelle Hal représente un atome d'halogène et G₁ représente un groupement choisi parmi -R_{c}, -C(W₂)-U-V, -C(W₂)-W₃-T₁, -S(O)ₙ-W₃-T₁ et -S(O)ₙ-T₁ dans lesquels R_{c}, W₂, W₃, U, V, T₁ et n sont tels que définis dans la formule (I), pour conduire aux composés de formule (I/m), cas particulier des composés de formule (I) : 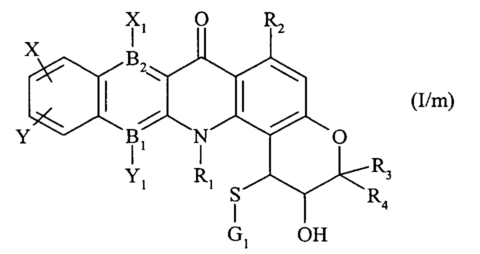 dans laquelle X, Y, X₁, Y₁, B₁, B₂, R₁, R₂, R₃, R₄, et G₁ sont tels que définis précédemment,
   composés de formule (I/m) qui peuvent être soumis à l'action d'un composé de formule (XVI) :

   Hal-G'₁ (XVI)

   dans laquelle Hal est tel que défini précédemment et G'₁ représente un groupement choisi parmi -R_{c}, -C(W₂)-U-V, -C(W₂)-W₃-T₁, -S(O)ₙ-W₃-T₁ et -S(O)ₙ-T₁ dans lesquels R_{c}, W₂, W₃, U, V, T₁ et n sont tels que définis dans la formule (I), pour conduire aux composés de formule (I/n), cas particulier des composés de formule (I) : 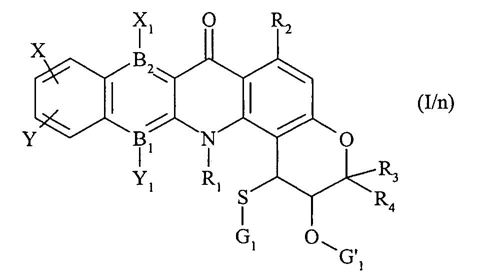 dans laquelle X, Y, X₁, Y₁, B₁, B₂, R₁, R₂, R₃, R₄, G₁ et G'₁ sont tels que définis précédemment,
γ) soit à des conditions réductrices en présence de NaBH₄, pour conduire aux composés de formule (I/o), cas particulier des composés de formule (I) : 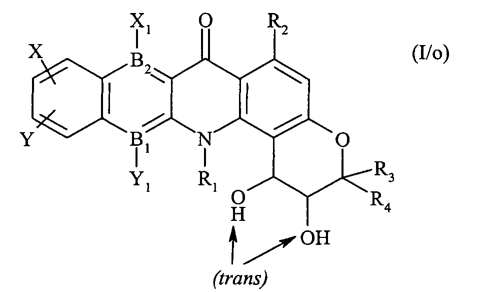
dans laquelle X, Y, X₁, Y₁, B₁, B₂, R₁, R₂, R₃, et R₄ sont tels que définis précédemment,
l'ensemble des composés de formules (*cis*-I/h) et (I/o) formant les composés de formule (I/p), 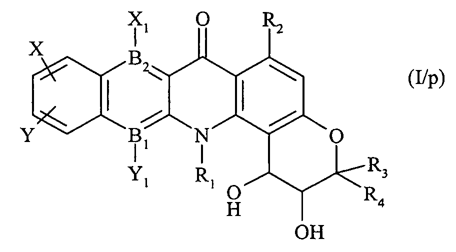 dans laquelle X, Y, X₁, Y₁, B₁, B₂, R₁, R₂, R₃, et R₄ sont tels que définis précédemment,

### composés de formule (I/p) pouvant être soumis :

α) soit à l'action d'un composé de formule (XVI) tel que défini précédemment, pour conduire aux composés de formule (I/q₁), (I/q₂) et (I/q₃), cas particuliers des composés de formule (I) : 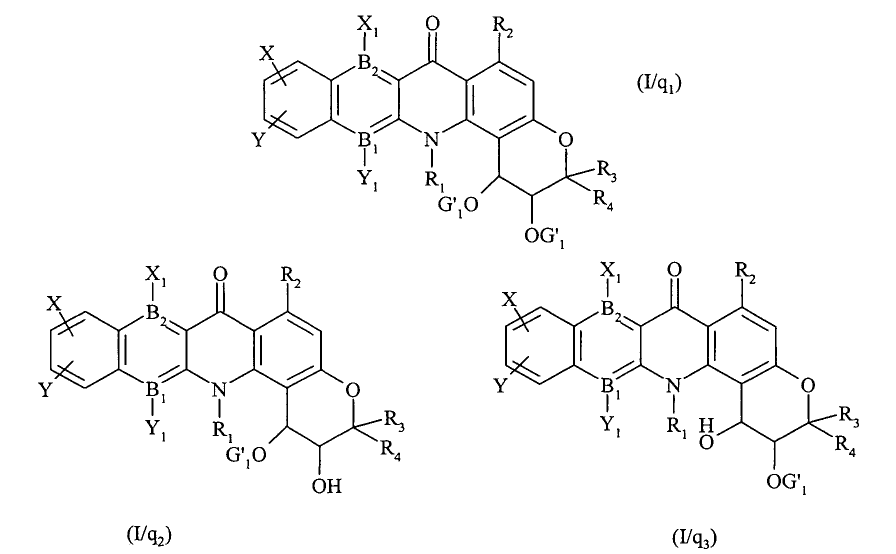 dans lesquelles X, Y, X₁, Y₁, B₁, B₂, R₁, R₂, R₃, R₄ et G'₁ sont tels que définis précédemment,

### composés de formules (I/q₂) et (I/q₃) qui sont éventuellement soumis :

***1)*** soit à l'action d'un alcool de formule R₃₀-OH dans laquelle R₃₀ représente un groupement alkyle (C₁-C₆) linéaire ou ramifié, pour conduire respectivement aux composés de formule (I/r₂) et (I/r₃), cas particuliers des composés de formule (I) : 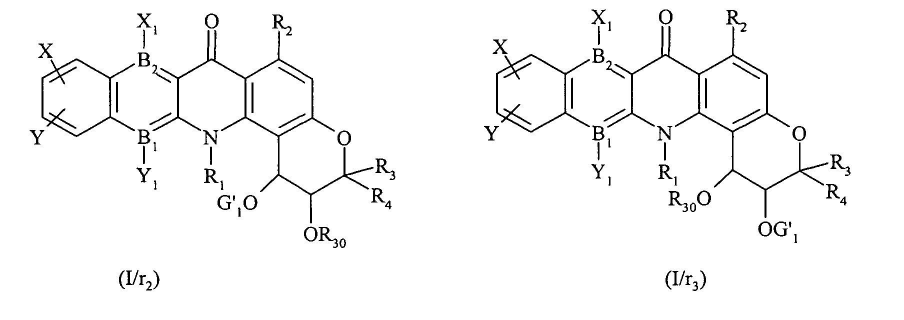 dans lesquelles X, Y, X₁, Y₁, B₁, B₂, R₁, R₂, R₃, R₄, G'₁ et R₃₀ sont tels que définis précédemment,
***2)*** soit à l'action d'un anhydride de formule (XVII) :

   [V-U-C(W₂)]₂O (XVII)

   dans laquelle W₂, U et V sont tels que définis dans la formule (I), pour conduire aux composés de formules (I/s₂) et (I/s₃), cas particuliers des composés de formule (I) : 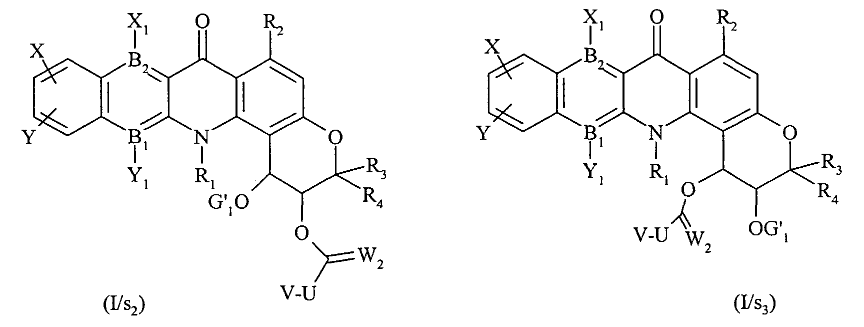 dans lesquelles X, Y, X₁, Y₁, B₁, B₂, *R*₁, R₂, R₃, R₄, G'₁, W₂, U et V sont tels que définis précédemment,
***3)*** soit à des conditions de déshydratation en milieu acide, pour conduire aux composés de formule (I/t₂) et (I/t₃), cas particuliers des composés de formule (I) : 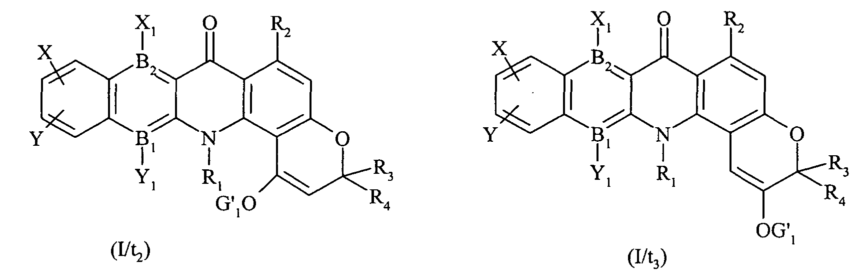 dans lesquelles X, Y, X₁, Y₁, B₁, B₂, R₁, R₂, R₃, R₄ et G'₁ sont tels que définis précédemment,
β) soit à l'action d'un composé de formules (XVIII) ou (XIX) 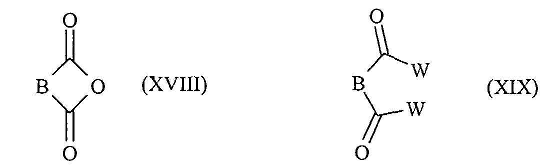 dans laquelle B est tel que défini dans la formule (I) et W représente un atome d'halogène ou un groupement hydroxyle, pour conduire aux composés de formule (I/u), cas particulier des composés de formule (I) : 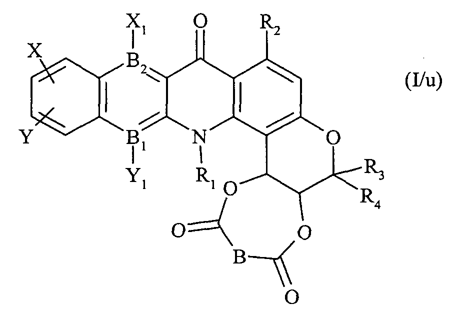 dans laquelle X, Y, X₁, Y₁, B₁, B₂, R₁, R₂, R₃, R₄ et B sont tels que définis précédemment,
γ) soit à l'action d'un dihalogénure d'alkyle (C₁-C₆) linéaire, pour conduire aux composés de formule (I/v), cas particulier des composés de formule (I): 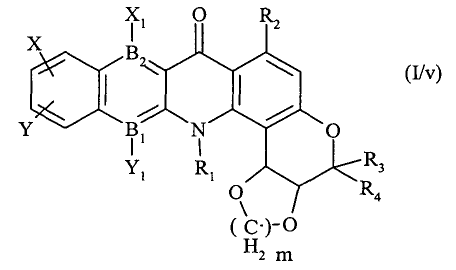 dans laquelle X, Y, X₁, Y₁, B₁, B₂, R₁, R₂, R₃ et R₄ sont tels que définis précédemment, et m est tel que défini dans la formule (I),
ε) soit à des conditions de déshydratation en milieu acide, pour conduire aux composés de formule (I/w), cas particulier des composés de formule (I) : 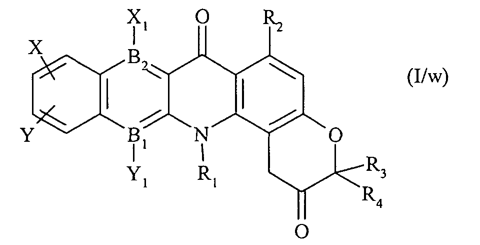 dans laquelle X, Y, X₁, Y₁, B₁, B₂, R₁, R₂, R₃ et R₄ sont tels que définis précédemment,
   composés de formule (I/w) qui sont éventuellement réduits en présence de NaBH₄, pour conduire aux composés de formule (I/x), cas particulier des composés de formule (I) : 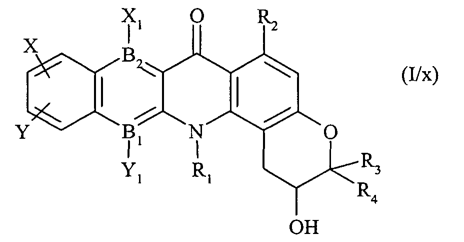 dans laquelle X, Y, X₁, Y₁, B₁, B₂, R₁, R₂, R₃ et R₄ sont tels que définis précédemment,
**d)** soit à l'action d'un péracide ou du diméthyle dioxirane, pour conduire aux composés de formule (I/y), cas particulier des composés de formule (I) : 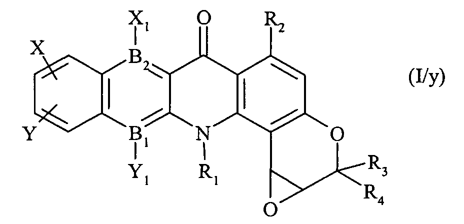 dans laquelle X, Y, X₁, Y₁, B₁, B₂, R₁, R₂, R₃ et R₄ sont tels que définis précédemment,
   composés de formule (I/y) qui sont éventuellement traités par de l'ammoniac ou une amine primaire ou secondaire pour conduire aux composés de formules (I/z₁) et (I/z₂), cas particuliers des composés de formule (I) : 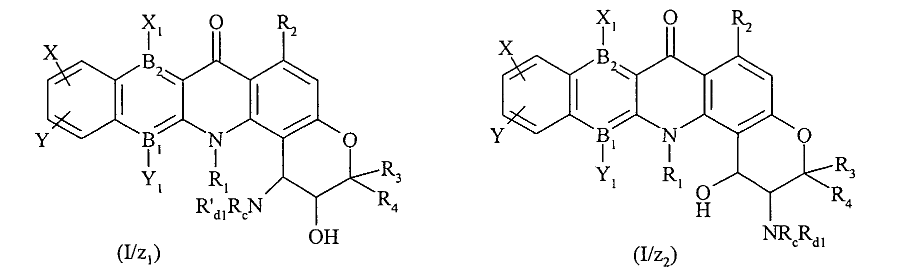 dans laquelle X, Y, X₁, Y₁, B₁, B₂, R₁, R₂, R₃ et R₄ sont tels que définis précédemment, R_{c} est tel que défini dans la formule (I) et R_{d1} représente un groupement choisi parmi R_{d}, -C(W₂)-U-V, -C(W₂)-W₃-T₁, -S(O)ₙ-W₃-T₁ et -S(O)ₙ-T₁ dans lesquels R_{d}, W₂, W₃, U, V et T₁ sont tels que définis précédemment,

### composés de formules (I/z₁) et (I/z₂) pouvant être soumis

α) soit, dans le cas où R_{c} et R_{d1} représentent un atome d'hydrogène, à l'action d'un composé de formule (XIII) tel que défini précédemment, pour conduire aux composés de formules (I/aa₁) et (I/aa₂), cas particuliers des composés de formule (I) : 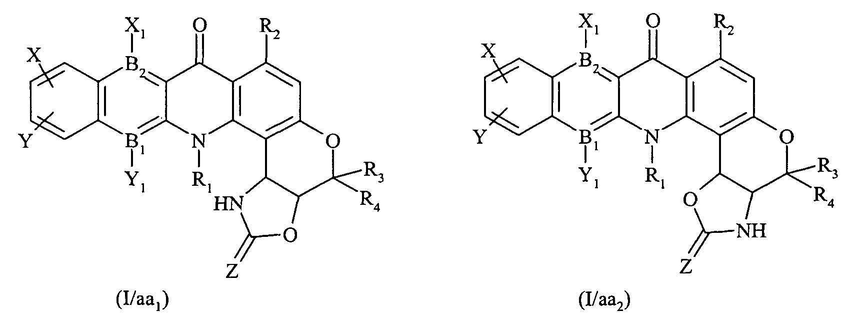 dans lesquelles X, Y, X₁, Y₁, B₁, B₂, R₁, R₂, R₃, R₄ et Z sont tels que définis précédemment,
β) soit à l'action d'un composé de formule (XIX) tel que défini précédemment, pour conduire aux composés de formules (I/ab₁) et (I/ab₂), cas particuliers des composés de formule (I) : 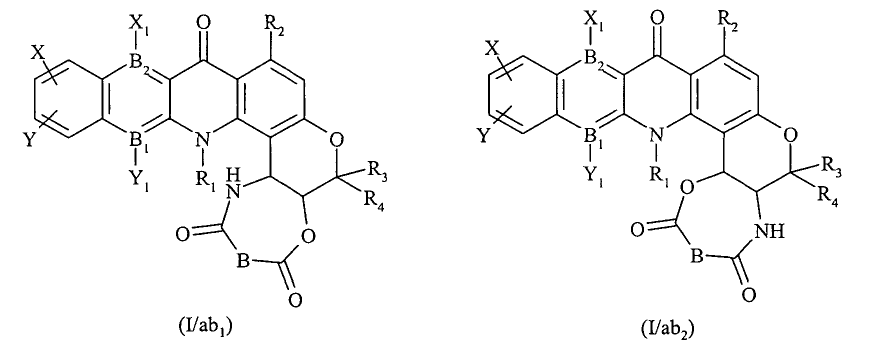 dans lesquelles X, Y, X₁, Y₁, B₁, B₂, R₁, R₂, R₃, R₄ et B sont tels que définis précédemment,
γ) soit à l'action d'un composé de formule (XVI) tel que défini précédemment, pour conduire aux composés de formules (I/ac₁) et (I/ac₂), cas particuliers des composés de formule (I) : 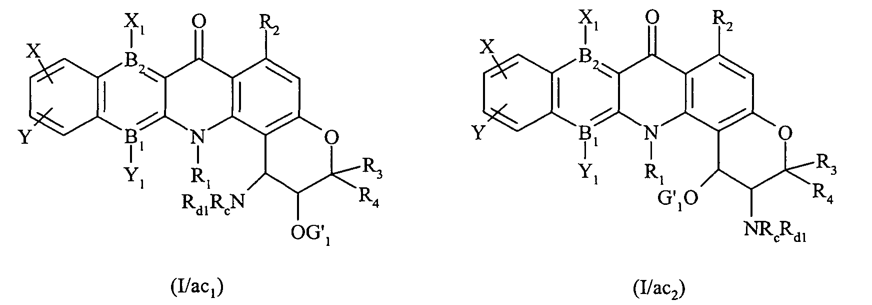 dans lesquelles X, Y, X₁, Y₁, B₁, B₂, R₁, R₂, R₃, R₄, R_{c}, R_{d1} et G'₁ sont tels que définis précédemment,
δ) soit à l'action de dibromure de triphénylphosphine en présence de triéthylamine pour conduire aux composés de formules (I/ad), cas particulier des composés de formule (I) : 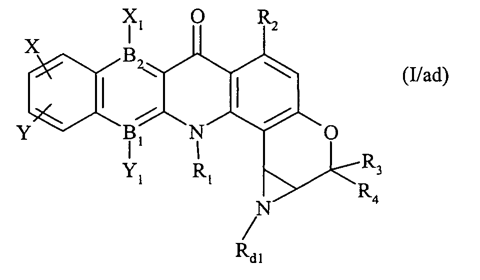 dans laquelle X, Y, X₁, Y₁, B₁, B₂, R₁, R₂, R₃, R₄ et R_{d1} sont tels que définis précédemment,
ε) soit à l'action de NaN₃ en présence d'eau oxygénée, suivi d'une étape de réduction, pour conduire aux composés de formules (I/ae), cas particulier des composés de formule (I) : 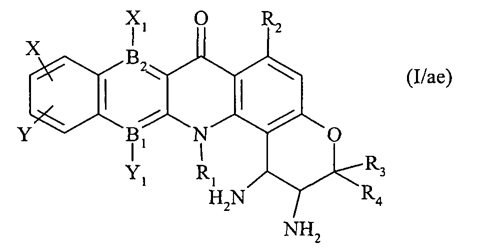 dans laquelle X, Y, X₁, Y₁, B₁, B₂, R₁, R₂, R₃ et R₄ sont tels que définis précédemment,

### composés de formule (I/ae) qui sont éventuellement soumis

***1)*** soit à l'action du dioxyde de carbone, en présence de diphényl phosphite, pour conduire aux composés de formule (I/af), cas particulier des composés de formule (I) : 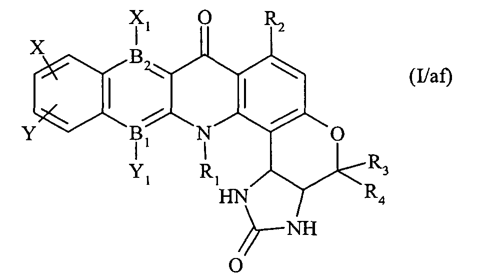 dans laquelle X, Y, X₁, Y₁, B₁, B₂, R₁, R₂, R₃ et R₄ sont tels que définis précédemment,
***2)*** soit à l'action d'un composé de formule (XVI) tel que défini précédemment, pour conduire aux composés de formules (I/ag₁), (I/ag₂) et (I/ag₃), cas particulier des composés de formule (I) : 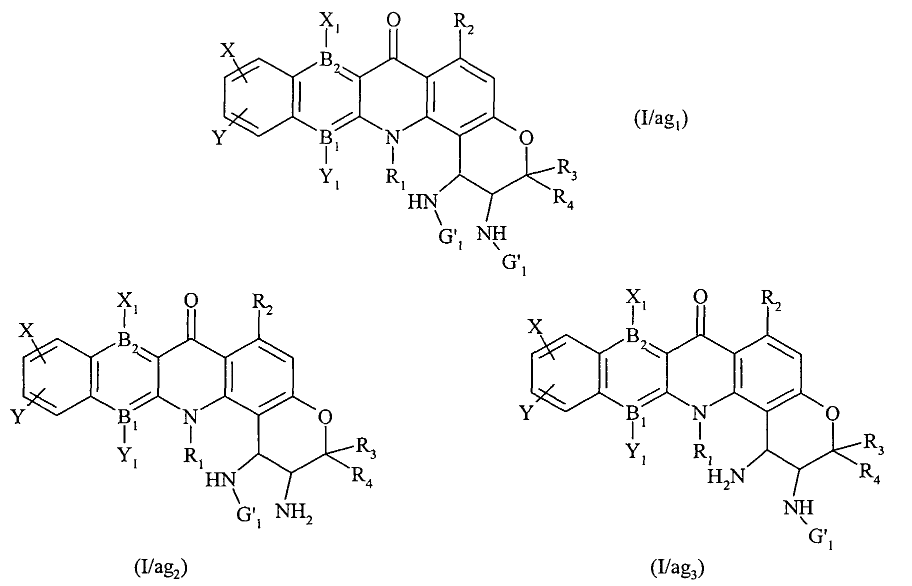 dans laquelle X, Y, X₁, Y₁, B₁, B₂, R₁, R₂, R₃, R₄ et G'₁ sont tels que définis précédemment,
   composés de formules (I/ag₂) et (I/ag₃) dont la fonction amine primaire est protégée par un groupement protecteur des groupes amines primaires pour conduire aux composés de formules (XX/a) et (XX/b), 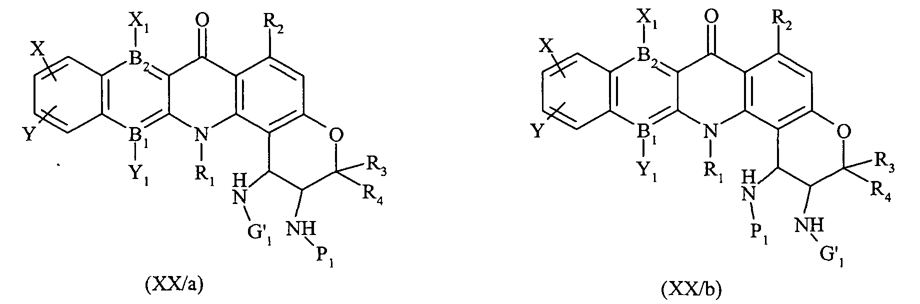 dans laquelle X, Y, X₁, Y₁, B₁, B₂, R₁, R₂, R₃ R₄, G'₁ sont tels que définis précédemment et P₁ représente un groupement protecteur de fonctions amines primaires,
   composés de formules (I/ag₁) et (XX/a) et (XX/b) qui sont éventuellement soumis à l'action d'un composé de formule (XXI) :

   R_{c1}-Hal (XXI)

   dans laquelle Hal représente un halogène et R_{c1} représente un groupement choisi parmi alkyle (C₁-C₆) linéaire ou ramifié, aryle ou arylalkyle (C₁-C₆) linéaire ou ramifié, puis qui sont soumis, dans le cas de composés de formule (XX/a) et (XX/b), à des conditions de déprotection de la fonction amine primaire, pour conduire aux composés de formules (I/ah₁), (I/ah₂) et (I/ah₃), cas particuliers des composés de formule (I) : 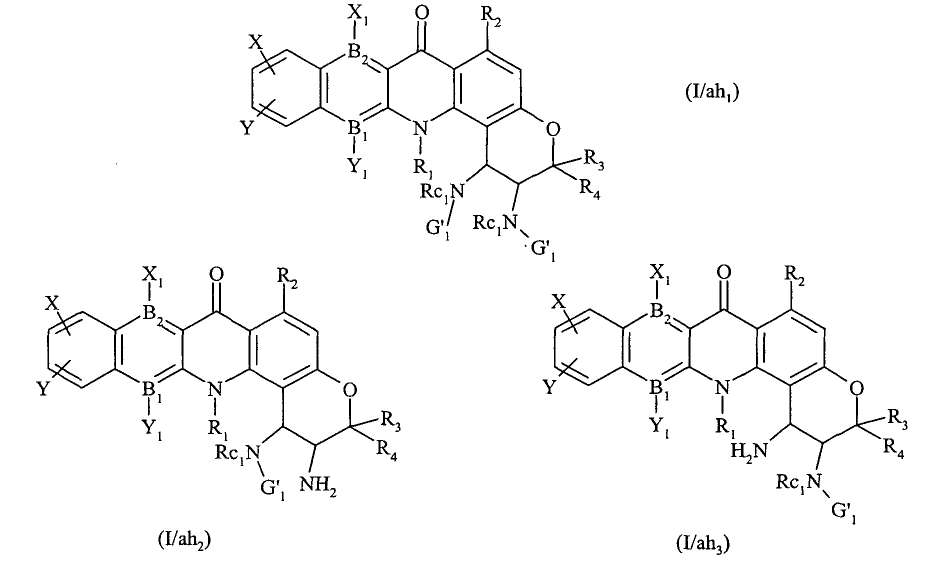 dans laquelle X, Y, X₁, Y₁, B₁, B₂, R₁, R₂, R₃ R₄, G'₁ et R_{c1} sont tels que définis précédemment,
   composés de formules (I/ah₂) et (I/ah₃) qui sont éventuellement soumis successivement à l'action d'un composé de formule (XXI) tel que défini précédemment puis d'un composé de formule (XXII) :

   R_{d'1}-Hal (XXII)

   dans laquelle Hal est tel que défini précédemment et R_{d'1} peut prendre les même définitions que R_{c1}, pour conduire aux composés de formules (I/ai₂) et (I/ai₃), cas particuliers des composés de formule (I) : 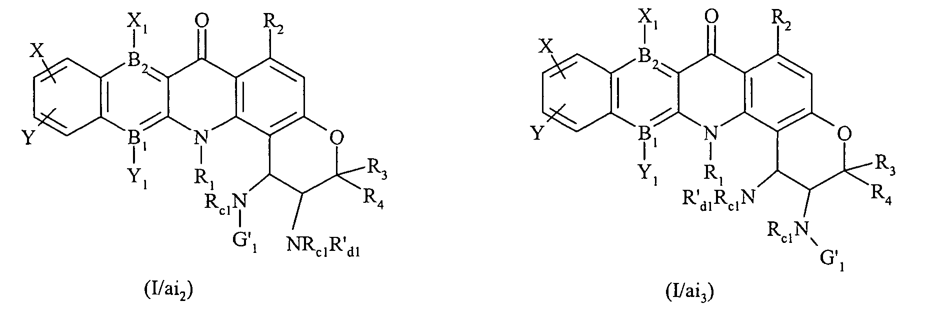 dans laquelle X, Y, X₁, Y₁, B₁, B₂, R₁, R₂, R₃, R₄, G'₁, R_{c1} et R_{d1} sont tels que définis précédemment,
φ) soit à des conditions de déshydratation en milieu acide, pour conduire aux composés de formules (I/aj₂) et (I/aj₃), cas particuliers des composés de formule (I) : 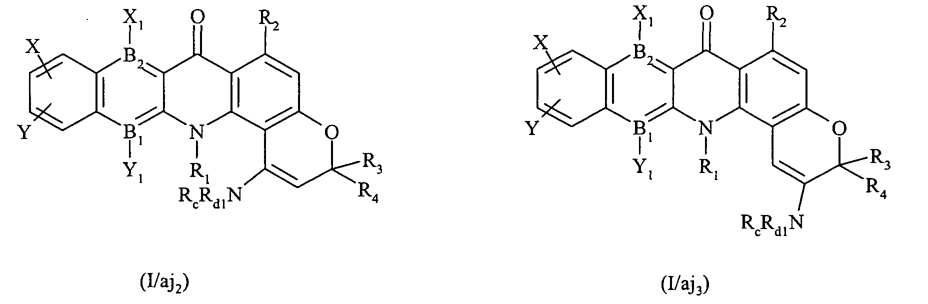 dans laquelle X, Y, X₁, Y₁, B₁, B₂, R₁, R₂, R₃, R₄, R_{c} et R_{d1} sont tels que définis précédemment,
**e)** soit à l'action d'un composé de formule (XXIII) :

   Hal-C(W₂)-T₁ (XXIII)

   dans laquelle Hal représente un atome d'halogène et W₂ et T₁ sont tels que définis dans la formule (I), pour conduire aux composés de formule (I/aj), cas particulier des composés de formule (I) : 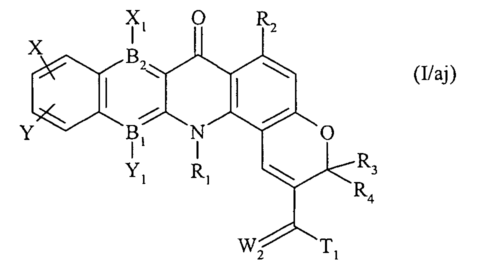 dans laquelle X, Y, X₁, Y₁, B₁, B₂, R₁, R₂, R₃ R₄, W₂ et T₁ sont tels que définis précédemment,
les composés (I/a) à (I/aj) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

Les composés de formules (II), (IV), (IX), (XII), (XIII), (XV), (XVI), (XVII), (XVIII), (XIX), (XXI) et (XXII) sont soit des produits commerciaux, soit obtenus selon des méthodes classiques de la synthèse organique bien connues de l'homme du métier.

Les composés de formule (I) présentent des propriétés antitumorales particulièrement intéressantes. Ils ont une excellente cytotoxicité in vitro sur des lignées cellulaires, issues de tumeurs murines et humaines, due à un blocage spécifique du cycle cellulaire, et sont actifs in vivo, chez la souris, sur des tumeurs transplantables murines et humaines. Les propriétés caractéristiques de ces composés permettent leur utilisation en thérapeutique en tant qu'agents antitumoraux.

La présente invention a également pour objet les compositions pharmaceutiques contenant comme principe actif au moins un composé de formule (I), ses énantiomères, diastéréoisomères, N-oxydes ou un de ses sels d'addition à une base ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 1000 mg en une ou plusieurs prises par jour.

Les exemples suivants illustrent l'invention mais ne la limitent en aucune façon.

Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion correspond à celui du produit salifié.

### PREPARATION 1 : Acide 2-chloro-3-quinoline carboxylique

5 g de 2-chloro-3-quinoline-carboxaldéhyde sont mis en solution dans 200 ml de diméthylformamide anhydre. 96 g de pyridinium dichromate sont ajoutés par petites portions à température ambiante, le ballon est surmonté d'une garde à chlorure de calcium et le mélange est maintenu sous agitation magnétique pendant 24 heures. Le milieu réactionnel est dilué par 1 litre d'eau et extrait avec du dichlorométhane. Les solutions organiques réunies sont évaporées à sec sous pression réduite, à froid, à l'aide d'une pompe à palette. Une chromatographie sur gel de silice (dichlorométhane puis dichlorométhane avec un gradient de méthanol de 0,1 à 2 %) permet d'isoler 1,53 g du produit attendu.
**Spectre de masse** **(DIC/NH**_{**3**}**) :** ***m*****/*****z*** **= 208 (M+ H)**^{**+**}**.**
**Point de fusion** **: 275°C**

### PREPARATION 2: 8-[(1,1-Diméthyl-2-propynyl)oxy]-10-hydroxy-6-méthyldibenzo[b,g][1,8]naphthyridin-11-(6H)-one

### Stade A : Acide 2-(3,5-diméthoxyanilino)-3-quinolinecarboxylique

Un échantillon de 5 g du composé de la préparation 1 et 4,93 g de 3,5-diméthoxyaniline sont chauffés à reflux dans 180 ml de diméthylformamide anhydre, en présence de 5,165 g de carbonate de potassium anhydre et 0,92 g de cuivre en poudre. Le milieu réactionnel, sous argon est chauffé à 155°C pendant 48 heures. Le milieu réactionnel est évaporé à sec, le résidu est extrait à plusieurs reprises par une solution d'hydroxyde de sodium 1 M. La solution est filtrée, puis acidifiée par l'acide chlorhydrique concentré jusqu'à pH = 6. Le précipité formé est filtré, bien lavé avec de l'eau, séché à l'air. Une chromatographie sur gel de silice (dichlorométhane puis dichlorométhane avec un gradient de méthanol de 0,1 à 2 %) permet d'isoler 2,98 g du produit attendu.
**Spectre de masse** **(ESI-SM) : 347 (M+ Na)**^{**+**}**, 325 (M+ H)**^{**+**}**.**
**Point de fusion** **: 215°C**

### Stade B : 8,10-Diméthoxydibenzo[b,g][1,8]naphthyridin-11(6H)-one

Un mélange de 5,6 g du composé du stade A précédent et 121,8 g d'acide polyphosphorique est chauffé à une température de 100°C pendant 2 heures. Le milieu réactionnel est versé sur de la glace, puis alcalinisé par une solution aqueuse d'hydroxyde de sodium à 30% jusqu'à pH = 6-7. Le précipité formé est filtré, lavé par de l'eau et séché à l'air. Une chromatographie sur gel de silice (dichlorométhane puis dichlorométhane avec un gradient de méthanol de 0,1 à 2 %) permet d'isoler 4,84 g du produit attendu.
**Spectre de masse** **(DIC/NH**_{**3**}**) : *m*/*z* = 307 (M+ H)**^{**+**}**.**
**Point de fusion** **: 279°C**

### Stade C : 8,10-Diméthoxy-6-méthyldibenzo[b,g][1,8]naphthyridin-11(6H)-one

A une solution de 4,5 g du composé du stade B précédent dans 250 ml d'acétone anhydre sont ajoutés 3,89 g d'hydroxyde de potassium anhydre en poudre. Le milieu réactionnel est chauffé à reflux à une température de 57°C sous argon pendant 15 min puis trois fois 10 ml d'iodométhane sont ajoutés toutes les 2 heures. Le milieu réactionnel est chauffé à reflux pendant 3 jours. Le solvant est évaporé sous pression réduite. Le résidu est extrait par l'eau, puis filtré. Une chromatographie sur gel de silice (dichlorométhane puis dichlorométhane avec un gradient de méthanol de 0,1 à 0,5 %), permet d'isoler 3,09 g du produit attendu.
**Spectre de masse** **(DIC/NH**_{**3**}**)** ***: m*****/*****z*** **= 321 (M+H)**^{**+**}**.**
**Point de fusion** **: 282°C**

### Stade D : 8,10-Dihydroxy-6-méthyldibenzo[b,g][1,8]naphthyridin-11(6H)-one

Un échantillon de 3g du composé du stade C précédent est mis en solution dans 150 ml d'acide bromhydrique (à 48 % dans H₂O) puis additionné de 50 ml d'acide acétique. L'ensemble est chauffé à reflux à 110°C pendant 3 jours. Le milieu réactionnel est versé sur de la glace, le précipité formé est filtré, lavé avec de l'eau et séché à l'air. Une chromatographie sur gel de silice (dichlorométhane puis dichlorométhane avec un gradient de méthanol de 0,1 à 2 %), permet d'isoler 2,35 g du produit attendu.
**Spectre de masse** **(DIC/NH**_{**3**}**) : m/z = 293 (M+ H)**^{**+**}**.**
**Point de fusion** **: 322°C**

### Stade E : 8-[(1,1-Diméthyl-2-propynyl)oxy]-10-hydroxy-6-méthyldibenzo[b,g][1,8] naphthyridin-11-(6H)-one

Une solution de 200 ml de diméthylformamide anhydre contenant 2 g du composé du stade D précédent, 5 g de 3-chloro-3-méthyl-1-butyne, 3 g de carbonate de potassium anhydre et 3 g d'iodure de potassium anhydre est chauffée à reflux à 70°C sous azote pendant 4 jours. Le milieu réactionnel est ensuite versé sur de la glace, le précipité est filtré, lavé avec de l'eau. La phase aqueuse est extraite avec du dichlorométhane (4 x 50 ml). Les phases organiques réunies sont séchées sur sulfate de sodium anhydre, filtrées et évaporées à sec. Une chromatographie sur gel de silice (dichlorométhane puis un gradient de dichlorométhane dans méthanol de 0,1 % à 0,4 %) permet d'isoler 972,8 mg du produit attendu.
**Spectre de masse** **(DIC/NH**_{**3**}**) : m/z = 359 (M+ H)**^{**+**}**.**
**Point de fusion** **: 218°C**

### PREPARATION 3 : 8-[(1,1-Diméthyl-2-propynyl)oxy]-6-méthyldibenzo[b,g][1,8] naphthyridin-11(6H)-one

Le produit est obtenu selon le procédé de la préparation 2 en utilisant la 3-méthoxyaniline à la place de la 3,5-diméthoxyaniline.

### PREPARATION 4 : 7,9-Dihydroxydibenzo[b,g][1,5]naphthyridin-6(11H)-one

A une solution de 5 g de carboxylate d'éthyl 3-amino-2-quinoline dans 50 ml d'heptane-1-ol sont additionnés 3,5 g de 1,3,5-trihydroxybenzène et 62,5 mg d'acide paratoluène sulfonique. Le mélange est maintenu sous agitation pendant 48 heures à reflux avec un Dean Stark, puis le mélange réactionnel est concentré sous vide. Une chromatographie sur gel de silice (cyclohexane/acétone : 90/10) permet d'isoler 5,2 g du produit attendu.

### PREPARATION 5: 3-[(1,1-Diméthyl-2-propynyl)oxy]-1-hydroxy-5-méthylquino [2,3-b]quinoxalin-12(5H)-one

Le produit est obtenu selon le procédé de la préparation 2 en utilisant l'acide 3-chloro-2-quinoxalinecarboxylique à la place de l'acide 2-chloro-3-quinolinecarboxylique.

### EXEMPLE 1 : 6-Hydroxy-3,3,14-triméthyl-3,14-dihydro-7H-benzo-[b]chroméno [6,5-g] [1,8]naphthyridin-7-one

Une solution de 500 mg du composé de la préparation 2 dans 170 ml de diméthylformamide anhydre est maintenu à reflux à 130°C sous azote pendant 2 jours. Le milieu réactionnel est évaporé sous pression réduite. Une chromatographie sur gel de silice (dichlorométhane puis dichlorométhane avec un gradient de méthanol de 0,1 à 0,3 %) permet d'isoler 248,86 mg du produit attendu.
**Spectre de masse** **(DIC/NH**_{**3**}**) :m/z = 359 (M+ H)**^{**+**}**.**
**Point de fusion** **: 278°C**

### EXEMPLE 2: 6-Méthoxy-3,3,14-triméthyl-3,14-dihydro-7H-benzo[b]chroméno [6,5-g][1,8]naphthyridin-7-one

840 mg d'hydrure de sodium (à 50 % dans l'huile de vaseline) sont ajoutés par petites portions à une solution, maintenue sous argon et à température ambiante, de 200 mg du composé de l'exemple 1 dans 40 ml de diméthylformamide anhydre. Le mélange ainsi obtenu est maintenu sous agitation à température ambiante et sous atmosphère inerte pendant 15 minutes, puis on ajoute 1,8 ml d'iodométhane. Le milieu réactionnel est chauffé à reflux à 52°C sous argon pendant 3 heures. On ajoute alors doucement de la glace, le précipité formé est filtré et séché à l'air. Une chromatographie sur gel de silice (dichlorométhane puis dichlorométhane avec un gradient de méthanol de 0,1 à 0,5 %) permet d'isoler 180,26 mg du produit attendu.
**Spectre de masse** **(DIC/NH**_{**3**}**) : *m*/*z* = 373 (M+ H)**^{**+**}**.**
**Point de fusion** **: 270°C**

### EXEMPLE 3 : (±)-cis-1,2-Dihydroxy-6-méthoxy-3,3,14-triméthyl-1,2,3,14-tétrahydro-7H-benzo[b]chroméno[6,5-g][1,8]naphthyridin-7-one

Un mélange de 500 mg du composé de l'exemple 2, de tétroxyde d'osmium (2,5 %) en solution dans 3,9 ml de 2-méthyl-2-propanol et de 626 mg de monohydrate de *N*-oxyde de 4-méthylmorpholine est mis en solution dans 40 ml d'un mélange de 55 ml *t*BuOH-THF-H₂O (10:3:1). Le milieu réactionnel est maintenu sous agitation à température ambiante pendant 3 jours. Une solution saturée de 55 ml de NaHSO₃ est alors ajoutée. Après agitation pendant une heure, le milieu réactionnel est extrait par de l'eau et du dichlorométhane. Les phases organiques réunies, séchées sur sulfate de sodium anhydre sont filtrées et évaporées à sec sous pression réduite. Une chromatographie sur gel de silice (dichlorométhane puis un gradient de méthanol dans le dichlorométhane de 0,1 à 2 %) permet d'isoler 314,29 mg du produit attendu.
**Spectre de masse** **(DIC/NH**_{**3**}**) : *m*/*z* = 407 (M+ H)**^{**+**}
**Point de fusion** **: 298°C**

### EXEMPLE 4 : (±)-cis-1,2-Diacétoxy-6-méthoxy-3,3,14-triméthyl-1,2,3,14-tétrahydro 7H-benzo[b]chroméno[6,5-g][1,8]naphthyridin-7-one

200 mg du composé de l'exemple 3 sont ajoutés à un mélange préalablement refroidi de 45 ml de pyridine anhydre, 11 ml d'anhydride acétique et de 4-diméthylamino-pyridine. Le milieu réactionnel est maintenu sous agitation à température ambiante pendant 1 jour, à l'abri de la lumière. Le milieu est ensuite versé sur de la glace, le précipité formé est filtré, lavé par de l'eau. La phase aqueuse est extraite avec du dichlorométhane. Les phases organiques réunies sont séchées sur sulfate de sodium anhydre, filtrées puis évaporées à sec sous pression réduite. Une chromatographie sur gel de silice (dichlorométhane puis un gradient de méthanol dans le dichlorométhane de 0,1 à 0,8 %) permet d'isoler 223 mg du produit attendu.
**Spectre de masse** **(DIC/NH**_{**3**}**) :** ***m*****/*****z*** **= 491 (M+ H)**^{**+**}
**Point de fusion** **: 244°C**

### EXEMPLE 5 : (±)-cis-7-Méthoxy-4,4,15-triméthyl-15,15c-dihydro-4H-benzo[b][1,3] dioxolo[4',5':3,4]chroméno[6,5-g][1,8]naphthyridin-2,8[3aH]-dione

A une solution de 200 mg du composé de l'exemple 3 dans 90 ml de 2-butanone sont additionnés 635,62 mg de *N,N'*-carbonyldiimidazole. Le milieu réactionnel est maintenu à reflux pendant 4 jours sous argon puis, après refroidissement, dilué par une solution aqueuse à 5 % de Na₂CO₃ (15 ml) et extrait avec de l'acétate d'éthyle. Les solutions organiques sont réunies, séchées sur sulfate de sodium anhydre, filtrées puis évaporées à sec sous pression réduite. Une chromatographie sur gel de silice (dichlorométhane puis un gradient de méthanol dans le dichlorométhane de 0,1 à 1,5 %) permet d'isoler 78 mg du produit attendu.
**Spectre de masse** **(ESI-SM) :** ***m*****/*****z*** **= 433 (M+H)**^{**+**}
**Point de fusion** **: 234°C**

### EXEMPLE 6: (±)-cis-1-{[(Diméthylamino)carbonyl]oxy}-6-méthoxy-3,3,14-triméthyl-7-oxo-2,3,7,14-tétrahydro-1H-benzo[b]chroméno[6,5-g] [1,8]naphthyridin-2-yl diméthylcarbamate

Ajouter à -10°C une solution de 0,123 mmol du composé de l'exemple 3 dans 4 ml de tétrahydrofurane anhydre à 0,698 mmol d'hydrure de potassium lavé à l'hexane. Après addition goutte à goutte à -10°C de 0,327 mmol de chlorure de N,N-diméthylcarbamoyle, l'agitation est maintenue 3h30 à température ambiante. Après addition de 50 ml d'acétate d'éthyle et de 10 ml d'une solution saturée en NaHCO₃, la phase organique est lavée à l'eau et séchée sur sulfate de magnésium puis évaporée sous pression réduite pour conduire au produit attendu.

### EXEMPLE 7: (±)-cis-6-Méthoxy-3,3,14-triméthyl-2-{[(4-méthylphényl)sulfonyl] oxy}-7-oxo-2,3,7,14-tétrahydro-1H-benzo[b]chroméno[6,5-g][1,8] naphthyridin-1-yl-4-méthylbenzènesulfonate

Le produit est obtenu selon le procédé de l'exemple 6 en utilisant le chlorure de tosyle à la place du chlorure de N,N-diméthylcarbamoyle.

### EXEMPLE 8 : Acide(±)-cis-4-{[1-(acétyloxy)-6-méthoxy-3,3,14-triméthyl-7-oxo-2,3,7,14-tétrahydro-1H-benzo[b]chroméno[6,5-g][1,8]naphthyridin-2-yl]oxy}-4-oxobutanoïque

A une solution de 0,5 mmol du composé de l'exemple 3 dans 3 ml de pyridine anhydre sont ajoutés 1,1 équivalents d'anhydride succinique et 1 mg de diméthylaminopyridine. Agiter 2 jours et dans l'obscurité à température ambiante puis ajouter 25 ml d'anhydride acétique et agiter 48 heures supplémentaires avant de concentrer sous pression réduite. Une chromatographie sur gel de silice (dichlorométhane/acide acétique : 99/1) permet d'isoler le produit attendu.

### EXEMPLE 9: Acide(±)-cis-5-{[(1-(acétyloxy)-6-méthoxy-3,3,14-triméthyl-7-oxo-2,3,7,14-tétrahydro-1H-benzo[b]chroméno[6,5-g][1,8]naphthyridin-2-yl]oxy}-5-oxopentanoïque

Le produit est obtenu selon le procédé de l'exemple 8 en utilisant l'anhydride glutarique à la place de l'anhydride succinique.

### EXEMPLE 10 : (±)-cis-1-(Acétyloxy)-6-methoxy-3,3,14-triméthyl-7-oxo-2,3,7,14-tétrahydro-1H-benzo[b]chroméno[6,5-g][1,8]naphthyridin-2-yl-[(tert-butoxycarbonyl)amino]acétate

Ajouter lentement 0,6 mmol de dicyclohexylcarbodiimide à une solution à 0°C de 0,5 mmol du composé de l'exemple 3 et 0,5 mmol d'acide 2-[(*tert*-butoxycarbonyl)amino] acétique dans 10 ml de diméthylformamide. Le milieu réactionnel est maintenu 5 heures à 0°C puis 16 heures à température ambiante. Après filtration et évaporation sous pression réduite, le résidu est mis en solution dans 2 ml de pyridine anhydre, additionné de 2 ml d'anhydride acétique, et agité à température ambiante et à l'obscurité pendant 48 heures. Après concentration sous pression réduite du milieu réactionnel, une chromatographie sur gel de silice du résidu (dichlorométhane) permet d'isoler le produit attendu.

### EXEMPLE 11 : (±)-cis-1-(Acétyloxy)-6-méthoxy-3,3,14-triméthyl-7-oxo-2,3,7,14-tétrahydro-1H-benzo[b]chroméno[6,5-g][1,8]naphthyridin-2-yl aminoacétate

Ajouter 0,14 µl d'iodotriméthylsilane à une solution à température ambiante de 0,1 mmol du composé de l'exemple 10 dans 1 ml de chloroforme. Le milieu réactionnel est agité 5 mn à température ambiante puis évaporé à sec sous pression réduite. Une chromatographie sur gel de silice (dichlorométhane/méthanol : 85/15) permet d'isoler le produit attendu.

### EXEMPLE 12 : 2-Butyryl-6-méthoxy-3,3,14-triméthyl-3,14-dihydro-7H-benzo[b] chroméno[6,5-g][1,8]naphthyridin-7-one

Un mélange de 0,81 mmol de chlorure de butyryle et 0,673 mmol d'AlCl₃ dans 2 ml de dichlorométhane anhydre est ajoutés par petites portions à 0,135 mmol du produit de l'exemple 2 dans 2 ml de dichlorométhane à 0°C. Le milieu réactionnel est agité 4 heures à température ambiante, puis versé sur une solution d'HCl à 10%. Après traitement classique des phases organiques et évaporation sous pression réduite de celles-ci, une chromatographie sur gel de silice du résidu (gradient dichlorométhane/méthanol) permet d'isoler le produit attendu.

### EXEMPLE 13 : (±)-cis Butyrate de 1-hydroxy-6-methoxy-3,3,14-triméthyl-7-oxo-2,3,7,14-tétrahydro-1H-benzo[b]chroméno[6,5-g][1,8]naphthyridin-2-yl

Additionner 2 équivalents de chlorure de butyryle à une solution de 0,74 mmol du composé de l'exemple 3 en présence de 4-diméthylaminopyridine dans 7 ml de pyridine anhydre. Agiter à température ambiante pendant 72 heures puis ajouter 5 équivalent de chlorure de butyryle et reprendre l'agitation 72 heures, puis évaporés à sec. Une chromatographie sur gel de silice permet d'isoler le produit attendu.

### EXEMPLE 14 : Butyrate de 6-méthoxy-3,3,14-triméthyl-7-oxo-7,14-dihydro-3H-benzo[b]chroméno[6,5-g][1,8]naphthyridin-2-yl

Ajouter 4 gouttes d'une solution d'HCl à 10% à une solution de 0,29 mmol du composé de l'exemple 13 dans 6 ml de dichlorométhane. Le milieu réactionnel est agité 3 jours à température ambiante, puis séché et concentré sous pression réduite. Une chromatographie sur gel de silice du résidu (gradient dichlorométhane/méthanol) permet d'isoler le produit attendu.

### EXEMPLE 15 : 2-Hydroxy-6-méthoxy-3,3,14-triméthyl-2,3-dihydro-1H-benzo[b] chroméno[6,5-g][1,8]naphthyridin-1,7(14H)-dione

Ajouter goutte à goutte pendant 30 minutes une suspension de 1,28 g de KMnO₄ dans 15 ml d'eau à une solution de 0,5 g du produit de l'exemple 2 dissous dans 25 ml d'acétone. Le milieu réactionnel est agité à température ambiante pendant 8 heures, puis après extraction et traitement classique le produit attendu est isolé par chromatographie sur gel de silice du résidu (dichlorométhane/méthanol : 98/2).

### EXEMPLE 16 : Acétate de 6-méthoxy-3,3,14-triméthyl-1,7-dioxo-2,3,7,14-tétrahydro-1H-benzo[b]chroméno[6,5-g][1,8]naphthyridin-2yl

Le produit est obtenu selon le procédé de l'exemple 13 à partir du composé de l'exemple 15 et en utilisant l'anhydride acétique à la place du chlorure de butyryle.

### EXEMPLE 17 : 3,3,14-Triméthyl-3,14-dihydro-7H-benzo-[b]chromeno[6,5-g][1,8] naphthyridin-7-one

Le produit est obtenu selon le procédé de l'exemple 1 en utilisant le composé de la préparation 3 à la place du composé de la préparation 2.

### EXEMPLE 18 : (±)-cis-1,2-Dihydroxy-3,3,14-triméthyl-1,2,3,14-tétrahydro-7H-benzo[b]chroméno[6,5-g][1,8]naphthyridin-7-one

Le produit est obtenu selon le procédé de l'exemple 3 en utilisant le composé de l'exemple 17 à la place du composé de l'exemple 2.

### EXEMPLE 19 : (±)-cis-1,2-(Diacétoxy)-3,3,14-triméthyl-7-oxo-1,2,3,14-tétrahydro-7H-benzo[b]chroméno[6,5-g][1,8]naphthyridin-7-one

Le produit est obtenu selon le procédé de l'exemple 4 en utilisant le composé de l'exemple 18 à la place du composé de l'exemple 3.

### EXEMPLE 20 : 6-Hydroxy-3,3-diméthyl-3,14-dihydro-7H-benzo[b]chroméno [5,6-g] [1,5]naphthyridin-7-one

A une solution de 2 g du produit de la préparation 4 dans 50 ml de diméthylformamide anhydre, sous atmosphère inerte, sont additionnés 2 g de carbonate de potassium anhydre. Après 15 minutes d'agitation à 65 °C, 2,4 g d'iodure de potassium anhydre et 4,4 g de 3-chloro-3-méthyl-1-butyne sont ajoutés et le milieu réactionnel est maintenu 24 heures à 65 °C puis 1 heure 30 à 130 °C. Après refroidissement, la solution est hydrolysée puis extraite au dichlorométhane. Les phases organiques rassemblées sont lavées à l'eau, puis par une solution de potasse 1M, séchées sur sulfate de sodium, puis évaporées. Une chromatographie sur gel de silice (cyclohexane/acétone : 90/10), permet d'isoler le produit attendu.

### EXEMPLE 21 : 6-Méthoxy-3,3,14-triméthyl-3,14-dihydro-7H-benzo[b]chroméno [5,6-g][1,5]naphthyridin-7-one

Le produit est obtenu selon le procédé de l'exemple 2 en utilisant le composé de l'exemple 20.

### EXEMPLE 22 : (±)-cis-1,2-Dihydroxy-6-méthoxy-3,3,14-triméthyl-1,2,3,14-tétrahydro-7H-benzo[b]chroméno[5,6-g][1,5]naphthyridin-7-one

Le produit est obtenu selon le procédé de l'exemple 3 en utilisant le composé de l'exemple 21.

### EXEMPLE 23 : (±)-cis-1,2-Diacétyloxy-6-méthoxy-3,3,14-triméthyl-1,2,3 14-tétrabydro-7H-benzo[b]chroméno[5,6-g][1,5]naphthyridin-7-one

Le produit est obtenu selon le procédé de l'exemple 4 en utilisant le composé de l'exemple 22.

### EXEMPLE 24 : 6-Hydroxy-3,3,14-triméthyl-3,14-dihydro-7H-pyrano[2',3':7,8]quino [2,3-b]quinoxalin-7-one

Le produit est obtenu selon le procédé de l'exemple 1 en utilisant le composé de la préparation 5 à la place du composé de la préparation 2.

### EXEMPLE 25 : 6-Méthoxy-3,3,14-triméthyl-3,14-dihydro-7H-pyrano[2',3':7,8]quino [2,3-b]quinoxalin-7-one

Le produit est obtenu selon le procédé de l'exemple 2 en utilisant le composé de l'exemple 24.

### EXEMPLE 26 : (±)-cis-1,2-Dihydroxy-6-méthoxy-3,3,14-triméthyl-1,2,3,14-tétrahydro-7H-pyrano[2',3':7,8]quino[2,3-b]quinoxalin-7-one

Le produit est obtenu selon le procédé de l'exemple 3 en utilisant le composé de l'exemple 25.

### EXEMPLE 27 : (±)-cis-1,2-Diacétoxy-6-méthoxy-3,3,14-triméthyl-1,2,3,4-tétrahydro-7H-pyrano[2',3':7,81]quino[2,3-b]quinoxalin-7-one

Le produit est obtenu selon le procédé de l'exemple 4 en utilisant le composé de l'exemple 26.

### EXEMPLE 28 : (±)-cis-7-Méthoxy-4, 4,15-triméthyl-15,15c-dihydro-4H-[1,3]dioxolo [4"5":4',5']pyrano[2',3':7,8]quino[2,3-b]quinoxaline-2,8(3aH)-dione

Le produit est obtenu selon le procédé de l'exemple 5 en utilisant le composé de l'exemple 26.

### ETUDE PHARMACOLOGIQUE DES COMPOSES DE L'INVENTION

### EXEMPLE 29 : Cytotoxicité in vitro

Deux lignées cellulaires ont été utilisées:
- 1 leucémie murine, L1210,
- 1 carcinome épidermoïde humain : KB-3-1

Les cellules sont cultivées dans du milieu RPMI 1640 complet contenant 10 % de sérum de veau foetal, 2 mM de glutamine, 50 unités/ml de pénicilline, 50 µg/ml de streptomycine et 10 mM d'Hepes, pH = 7,4. Les cellules sont réparties dans des microplaques et exposées aux composés cytotoxiques. Les cellules sont ensuite incubées pendant 2 jours (L1210) ou 4 jours (KB-3-1) dans un incubateur, à 37°C en présence de 5 % de CO₂. Le nombre de cellules viables est ensuite quantifié par un essai colorimétrique, le Microculture Tetrazolium Assay (Cancer Res. 1987, 47, 939-942).
Les résultats sont exprimés en IC₅₀, concentration en cytotoxique qui inhibe à 50 % la prolifération des cellules traitées. A titre d'exemple, le composé de l'exemple 4 présente respectivement une IC₅₀ de 0,32 µM sur L1210 et de 0,037 µM sur KB-3-1.

### EXEMPLE 30 : Composition pharmaceutique : soluté injectable

| | |
|---|---|
| Composé de l'exemple 4 | 10 mg |
| Eau distillée pour préparations injectables | 25 ml |

## Revendications

1. Composés de formule (I) : 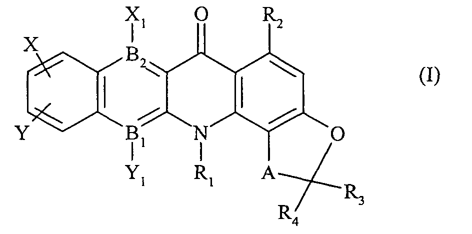 dans laquelle :
• B₁ et B₂, identiques ou différents, indépendamment l'un de l'autre, représentent chacun un atome de carbone ou un atome d'azote étant entendu qu'au moins un des deux groupements B₁ ou B₂ représente un atome d'azote,
• X et Y, identiques ou différents, indépendamment l'un de l'autre, représentent chacun un ou plusieurs groupements choisis parmi :
- atome d'hydrogène, halogène,
- groupement hydroxy, alkoxy (C₁-C₆) linéaire ou ramifié, nitro, cyano, alkyle (C₁-C₆) linéaire ou ramifié, alkényle (C₂-C₆) linéaire ou ramifié, polyhalogénoalkyle (C₁-C₆) linéaire ou ramifié, ou
- groupement de formule -NRₐR_{b} dans lequel:
Rₐ et R_{b}, identiques ou différents, indépendamment l'un de l'autre, représentent chacun un groupement choisi parmi atome d'hydrogène, -C(O)-CF₃, -C(O)-NH₂ ou groupement alkyle (C₁-C₆) linéaire ou ramifié, éventuellement substitué par un groupement NR'ₐR'_{b} dans lequel :
R'ₐ et R'_{b}, identiques ou différents, indépendamment l'un de l'autre, représentent chacun un groupement choisi parmi atome d'hydrogène, groupement alkyle (C₁-C₆) linéaire ou ramifié, ou R'ₐ et R'_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
ou Rₐ et R_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
• X₁ et Y₁, identiques ou différents, indépendamment l'un de l'autre, représentent chacun un groupement choisi parmi les substituants tels que définis pour X et Y,
étant entendu que lorsque B₁ et/ou B₂ représentent un atome d'azote, B₁ et B₂ ne portent pas de substituants Y₁ et X₁ respectivement,
• R₁ représente un atome d'hydrogène ou un groupement alkyle (C₁-C₆) linéaire ou ramifié,
• R₂ représente un groupement choisi parmi atome d'hydrogène, groupement alkyle (C₁-C₆) linéaire ou ramifié, -OR"ₐ; -NR'ₐR'_{b} ; O-Tₐ-OR"ₐ; -NR"ₐ-Tₐ-NR'ₐR'_{b}; -NR"ₐ-C(O)-TₐH ; O-C(O)-TₐH ; -O-Tₐ-NR'ₐR'_{b} ; -NR"ₐ-Tₐ-OR"ₐ; -NR"ₐ-Tₐ-CO₂R"ₐ ; -NR"ₐ-C(O)-Tₐ-NR'ₐR'_{b}, dans lesquels :
* Tₐ représente une chaîne alkylène (C₁-C₆) linéaire ou ramifiée,
* R'ₐ et R'_{b} sont tels que définis précédemment,
* R"ₐ représente un groupement choisi parmi atome d'hydrogène, groupement alkyle (C₁-C₆) linéaire ou ramifié,
• R₃ et R₄, identiques ou différents, indépendamment l'un de l'autre, représentent chacun un atome d'hydrogène ou un groupement alkyle (C₁-C₆) linéaire ou ramifié, ou R₃ et R₄ forment ensemble avec l'atome de carbone qui les portent, un cycle de 3 à 6 chaînons, monocyclique,
• A représente un groupement de formule:
a) -CH(R₅)-CH(R₆)- dans lequel :
* *R*_{*5*} *et R*_{*6*}*, identiques ou différents, indépendamment l'un de l'autre représentent chacun un groupement choisi parmi :*
1) atome d'hydrogène,
2) groupement OR_{c}, NR_{c}R_{d}, SR_{c}, dans lesquels :
- R_{c}, R_{d}, identiques ou différents, indépendamment l'un de l'autre, représentent chacun un groupement choisi parmi atome d'hydrogène, groupement alkyle (C₁-C₆) linéaire ou ramifié, ou un groupement aryle, arylalkyle (C₁-C₆) linéaire ou ramifié,
ou un groupement C(O)-Rₑ dans lequel Rₑ représente un groupement choisi parmi atome d'hydrogène, groupement aryle, ou NR"'ₐR"'_{b} dans lequel R"'ₐ et R"'_{b}, identiques, représentent un atome d'hydrogène ou R"'ₐ et R"'_{b} forment ensemble avec l'atome d'azote qui les portent, un hétérocycle de 5 à 7 chaînons, monocyclique, contenant éventuellement au sein du système cyclique un second hétéroatome choisi parmi oxygène et azote,
3) W₁-C(W₂)-U-V dans lequel :
α) W₁ représente un atome d'oxygène, de soufre ou NR_{c} (dans lequel R_{c} est tel que défini précédemment),
β) W₂ représente un atome d'oxygène ou un atome de soufre,
γ) U représente une chaîne alkylène (C₁-C₈) linéaire ou ramifiée ou une chaîne alkénylène (C₂-C₈) linéaire ou ramifiée,
δ) V représente un groupement choisi parmi :
- atome d'hydrogène,
- groupement aryle,
- groupements OR_{c}, CO₂R_{c}, COR_{c}, CONR'ₐR'_{b}, NR_{c}R_{d}, N(R_{c})-CO₂R'_{c}, N(R_{c})-COR'_{c}, dans lesquels R'ₐ, R'_{b}, R_{c} et R_{d} sont tels que définis précédemment et R'_{c} représente un groupement choisi parmi atome d'hydrogène, groupement alkyle (C₁-C₆) linéaire ou ramifié, ou un groupement aryle, arylalkyle (C₁-C₆) linéaire ou ramifié,
ε) U représente une liaison lorsque W₂ ne représente pas un atome d'oxygène et lorsque V ne représente pas un groupement choisi parmi:
- atome d'hydrogène,
- groupement aryle,
- NH₂,
4) W₁-C(W₂)-W₃-T₁ dans lequel :
α) W₁ et W₂ sont tels que définis précédemment,
β) W₃ représente un atome d'oxygène, de soufre ou NR_{c} dans lequel R_{c} est tel que défini précédemment,
γ) T₁ représente un groupement choisi parmi :
- atome d'hydrogène,
- alkyle (C₁-C₆) linéaire ou ramifié,
- alkényle (C₂-C₆) linéaire ou ramifié,
- aryle, arylalkyle (C₁-C₆) linéaire ou ramifié,
- chaîne alkylène (C₁-C₆) linéaire ou ramifiée ou une chaîne alkénylène (C₂-C₆) linéaire ou ramifiée, chacune étant substituée par un groupement OR_{c}
dans lequel R_{c} est tel que défini précédemment ou NR'ₐR'_{b} dans lequel R'ₐ et R'_{b} sont tels que définis précédemment,
5) W₁-S(O)ₙW₃-T₁ dans lequel :
α) W₁, W₃ et T₁ sont tels que définis précédemment,
P) n représente un nombre entier choisi parmi 1 et 2,
6) W₁-S(O)ₙ-T₁ dans lequel W₁, T₁ et n sont tels que définis précédemment,
7) C(W₂)-T₁ dans lequel W₂ et T₁ sont tels que définis précédemment,
* *ou R*_{*5*} *et R*_{*6*}*, forment ensemble :*
1) un groupement 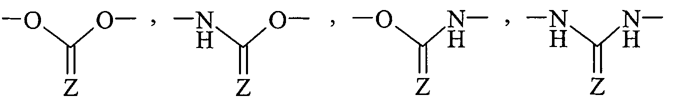 dans lesquels Z représente un atome d'oxygène ou un atome de soufre,
2) un groupement -O-(CH₂)ₘ-O- dans lequel m représente un nombre entier compris entre 1 et 4 inclus,
3) un groupement 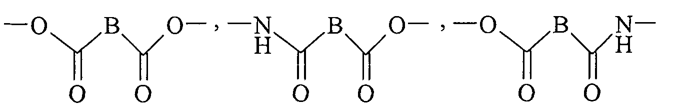 dans lesquels B représente une liaison simple, une chaîne alkylène (C₁-C₆) linéaire ou ramifiée ou une chaîne alkénylène (C₂-C₆) linéaire ou ramifiée,
* *ou R*_{*5*} *et R*_{*6*}*, forment ensemble avec les atomes de carbone qui les portent, un groupement oxirane ou un groupement aziridine,* éventuellement substitué sur l'atome d'azote par un groupement alkyle (C₁-C₆) linéaire ou ramifié,
b) -CH=C(R₇)- ou -C(R₇)=CH- dans lesquels R₇ représente un groupement choisi parmi :
- atome d'hydrogène,
- groupement OR"ₐ, W₁-C(W₂-U-V, W₁-C(W₂)-W₃-T₁, W₁-S(O)ₙ-W₃-T₁, W₁-S(O)ₙ-T₁ ou C(W₂)-T₁ dans lesquels R"ₐ, W₁, W₂, U, V, W₃, T₁ et n sont tels que définis précédemment,
c) -C(O)-CH(R₈)- ou - CH(R₈)-C(O)- dans lesquels R₈ représente un groupement choisi parmi :
- atome d'hydrogène,
- alkylcarbonyloxy (C₁-C₆) linéaire ou ramifié, groupement OR"ₐ dans lequel R"ₐ est tel que défini précédemment,
leurs énantiomères, diastéréoisomères, N-oxydes, ainsi que leurs sels d'addition à un acide ou à une base pharmaceutiquement acceptable,
étant entendu que par aryle, on comprend un groupement phényle ou naphtyle, comportant éventuellement une ou plusieurs substitutions, identiques ou différentes, choisies parmi alkyle (C₁-C₆) linéaire ou ramifié, alkényle (C₂-C₆) linéaire ou ramifié, hydroxy, halogène, carboxy, nitro, amino, alkylamino ou dialkylamino (C₁-C₆) linéaire ou ramifié, alkoxy (C₁-C₆) linéaire ou ramifié, acyle (C₁-C₆) linéaire ou ramifié, et alkylcarbonyloxy (C₁-C₆) linéaire ou ramifié,
parmi les hétérocycles de 5 à 7 chaînons, monocyclique, contenant éventuellement au sein du système cyclique un second hétéroatome choisi parmi oxygène et azote, on peut citer à titre non limitatif les groupements pyrrolidinyle, isoxazolidinyle, oxazolidinyle, pyrazolidinyle, imidazolidinyle, pipéridinyle, morpholinyle, hexahydropyridiazinyle, hexahydropyrimidinyle, pipérazinyle, azépanyle, oxazépanyle, diazépanyle,
parmi les cycles de 3 à 6 chaînons, monocyclique, on peut citer à titre non limitatif les groupements cyclopropyle, cyclobutyle, cyclopentyle et cyclohexyle.

2. Composés de formule (I) selon la revendication 1, **caractérisés en ce qu'**ils représentent des composés de formule (IA) : 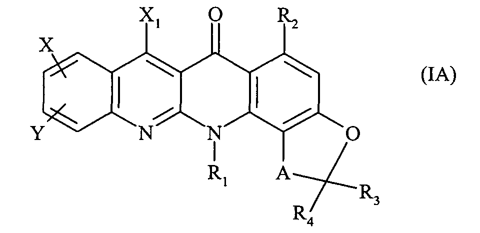 dans laquelle X, Y, X₁, R₁, R₂, R₃, R₄ et A sont tels que définis dans la formule (I), leurs énantiomères, diastéréoisomères, N-oxydes, ainsi que leurs sels d'addition à un acide ou à une base pharmaceutiquement acceptable.

3. Composés de formule (I) selon l'une quelconque des revendications 1 ou 2, **caractérisés en ce qu'**ils représentent des composés de formule (IB) : 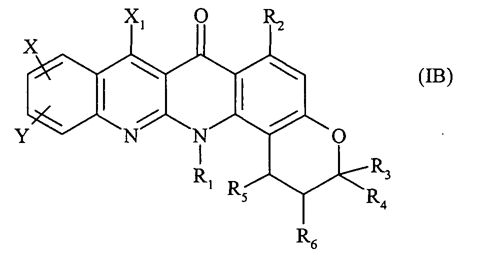 dans laquelle X, Y, X₁, R₁, R₂, R₃, R₄, R₅ et R₆ sont tels que définis dans la formule (I), leurs énantiomères, diastéréoisomères, N-oxydes, ainsi que leurs sels d'addition à un acide ou à une base pharmaceutiquement acceptable.

4. Composés de formule (I) selon la revendication 1, **caractérisés en ce qu'**ils représentent des composés de formule (IC) : 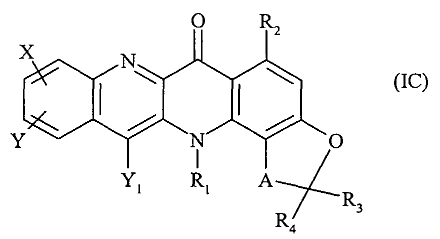 dans laquelle X, Y, Y₁, R₁, R₂, R₃ R₄ et A sont tels que définis dans la formule (I), leurs énantiomères, diastéréoisomères, N-oxydes, ainsi que leurs sels d'addition à un acide ou à une base pharmaceutiquement acceptable.

5. Composés de formule (I) selon l'une quelconque des revendications 1 ou 4, **caractérisés en ce qu'**ils représentent des composés de formule (ID) : 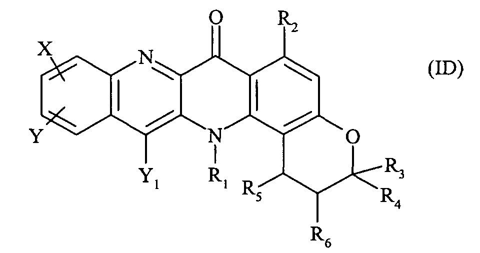 dans laquelle X, Y, Y₁, R₁, R₂, R₃, R₄, R₅ et R₆ sont tels que définis dans la formule (I), leurs énantiomères, diastéréoisomères, N-oxydes, ainsi que leurs sels d'addition à un acide ou à une base pharmaceutiquement acceptable.

6. Composés de formule (I) selon la revendication 1, **caractérisés en ce qu'**ils représentent des composés de formule (IE) : 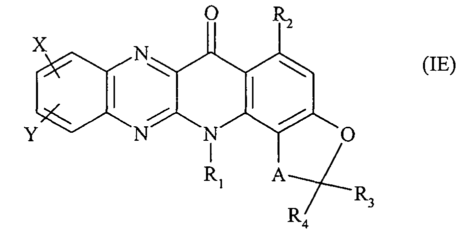 dans laquelle X, Y, R₁, R₂, R₃, R₄ et A sont tels que définis dans la formule (I), leurs énantiomères, diastéréoisomères, N-oxydes ainsi que leurs sels d'addition à un acide ou à une base pharmaceutiquement acceptable.

7. Composés de formule (I) selon l'une quelconque des revendications 1 ou 6, **caractérisés en ce qu'**ils représentent des composés de formule (IF) : 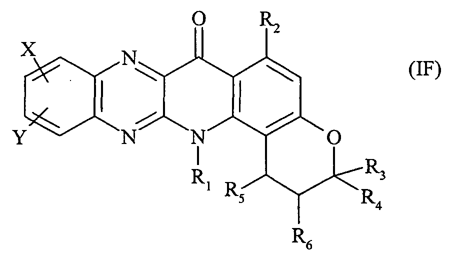 dans laquelle X, Y, R₁, R₂, R₃, R₄, R₅ et R₆ sont tels que définis dans la formule (I), leurs énantiomères, diastéréoisomères, N-oxydes ainsi que leurs sels d'addition à un acide ou à une base pharmaceutiquement acceptable.

8. Composés de formule (I) selon la revendication 1, **caractérisés en ce qu'**ils représentent des composés de formule (IB), (ID) et (IF) dans lesquels R₅ et R₆, identiques, représentent un groupement de formule -OR_{c}, W₁-C(W₂)-U-V ou R₅ et R₆ forment ensemble un groupement 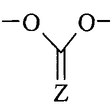 dans lesquels R_{c}, W₁, W₂, U, V et Z sont tels que définis dans la formule (I), leurs énantiomères, diastéréoisomères, N-oxydes ainsi que leurs sels d'addition à un acide ou à une base pharmaceutiquement acceptable.

9. Composés de formule (I) selon l'une quelconque des revendications 1 ou 8, **caractérisés en ce qu'**ils représentent des composés de formules (IB), (ID) et (IF) dans lesquels R₅ et R₆, identiques, représentent un groupement de formule -OR_{c} dans laquelle R_{c} représente un atome d'hydrogène ou un groupement alkyle (C₁-C₆) linéaire ou ramifié, leurs énantiomères, diastéréoisomères, N-oxydes ainsi que leurs sels d'addition à un acide ou à une base pharmaceutiquement acceptable.

10. Composés de formule (I) selon l'une quelconque des revendications 1 ou 8, **caractérisés en ce qu'**ils représentent des composés de formules (IB), (ID) et (IF) dans lesquels R₅ et R₆, identiques, représentent un groupement de formule W₁-C(W₂)-U-V dans laquelle W₁ et W₂ représentent un atome d'oxygène, U représente une chaîne alkylène (C₁-C₆) linéaire ou ramifié et V représente un atome d'hydrogène, leurs énantiomères, diastéréoisomères, N-oxydes ainsi que leurs sels d'addition à un acide ou à une base pharmaceutiquement acceptable.

11. Composés de formule (I) selon l'une quelconque des revendications 1 ou 8, **caractérisés en ce qu'**ils représentent des composés de formules (IB), (ID) et (IF) dans lesquels R₅ et R₆ forment ensemble un groupement 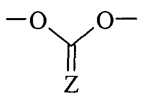 dans lequel Z représente un atome d'oxygène, leurs énantiomères, diastéréoisomères, N-oxydes ainsi que leurs sels d'addition à un acide ou à une base pharmaceutiquement acceptable.

12. Composés de formule (I) selon l'une quelconque des revendications 1 ou 2, **caractérisés en ce qu'**ils représentent des composés de formule (IG) : 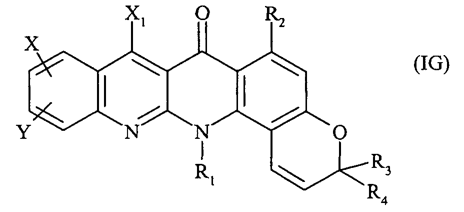 dans laquelle X, Y, X₁, R₁, R₂, R₃ et R₄ sont tels que définis dans la formule (I), leurs énantiomères, diastéréoisomères, N-oxydes ainsi que leurs sels d'addition à un acide ou à une base pharmaceutiquement acceptable.

13. Composés de formule (I) selon l'une quelconque des revendications 1 ou 4, **caractérisés en ce qu'**ils représentent des composés de formule (IH) : 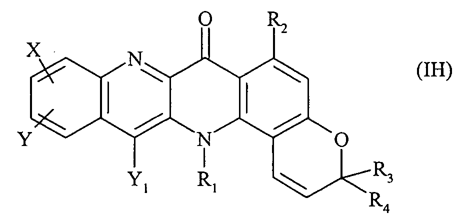 dans laquelle X, Y, Y₁, R₁, R₂, R₃ et R₄ sont tels que définis dans la formule (I), leurs énantiomères, diastéréoisomères, N-oxydes ainsi que leurs sels d'addition à un acide ou à une base pharmaceutiquement acceptable.

14. Composés de formule (I) selon l'une quelconque des revendications 1 ou 6, **caractérisés en ce qu'**ils représentent des composés de formule (IJ) : 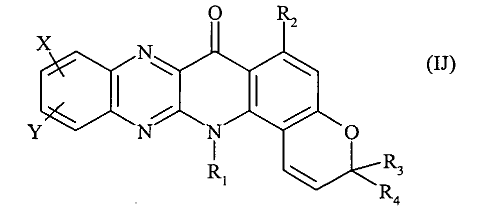 dans laquelle X, Y, R₁, R₂, R₃ et R₄ sont tels que définis dans la formule (I), leurs énantiomères, diastéréoisomères, N-oxydes ainsi que leurs sels d'addition à un acide ou à une base pharmaceutiquement acceptable.

15. Composés de formule (I) selon l'une quelconque des revendications 1 à 14, **caractérisés en ce que** R₃ et R₄ représentent un groupement alkyle (C₁-C₆) linéaire ou ramifié, leurs énantiomères, diastéréoisomères, N-oxydes ainsi que leurs sels d'addition à un acide ou à une base pharmaceutiquement acceptable.

16. Composés de formule (I) selon l'une quelconque des revendications 1 à 14, **caractérisés en ce que** R₂ représente un groupement -OR"ₐ dans lequel R"ₐ est tel que défini dans la formule (I), leurs énantiomères, diastéréoisomères, N-oxydes ainsi que leurs sels d'addition à un acide ou à une base pharmaceutiquement acceptable.

17. Composés de formule (I) selon l'une quelconque des revendications 1 à 14 et 16, **caractérisés en ce que** R₂ représente un groupement -OR"ₐ dans lequel R"ₐ représente un groupement alkyle (C₁-C₆) linéaire ou ramifié, et encore plus préférentiellement un groupement méthyl, leurs énantiomères, diastéréoisomères, N-oxydes ainsi que leurs sels d'addition à un acide ou à une base pharmaceutiquement acceptable.

18. Composés de formule (I) selon l'une quelconque des revendications 1 à 14, **caractérisés en ce que** X, Y, X₁ et Y₁ représentent un atome d'hydrogène, leurs énantiomères, diastéréoisomères, N-oxydes ainsi que leurs sels d'addition à un acide
ou à une base pharmaceutiquement acceptable.

19. Composés de formule (I) selon la revendication 1 qui sont le:
- (±)-*cis*-1,2-dihydroxy-6-méthoxy-3,3,14-triméthyl-1,2,3,14-tétrahydro-7*H*-benzo[*b*] chroméno[6,5-*g*][1,8]naphthyridin-7-one,
- (±)-*cis*-1,2-diacétoxy-6-méthoxy-3,3,14-triméthyl-1,2,3,14-tétrahydro-7*H*-benzo[*b*] chroméno[6,5-*g*][1,8]naphthyridin-7-one,
- (±)-*cis*-7-méthoxy-4,4,15-triméthyl-15,15c-dihydro-4*H*-benzo[*b*][1,3]dioxolo [4',5':3,4]chroméno[6,5-*g*][1,8]naphthyridine-2,8(3a*H*)-dione,
leurs énantiomères, diastéréoisomères, N-oxydes ainsi que leurs sels d'addition à un acide ou à une base pharmaceutiquement acceptable.

20. Procédé de préparation des composés de formule (I) selon la revendication 1, **caractérisés en ce qu'**on utilise comme produit de départ un composé de formule (II) : 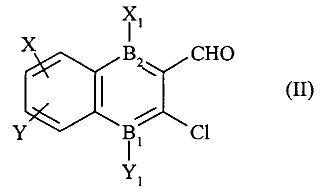 dans laquelle X, Y, X₁, Y₁, B₁ et B₂ sont tels que définis dans la formule (I),
composés de formule (II) qui sont mis en présence de pyridinium dichromate pour conduire aux composés de formule (III) : 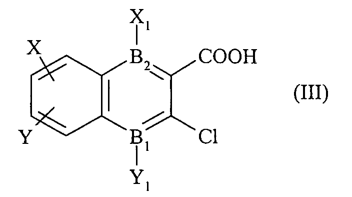 dans laquelle X, Y, X₁, Y₁, B₁ et B₂ sont tels que définis dans la formule (I),
composés de formule (III) qui sont traités en milieu basique et anhydre par un composé de formule (IV) : 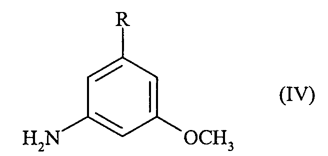 dans laquelle R représente un atome d'hydrogène, un groupement hydroxy, un groupement alkyle (C₁-C₆) linéaire ou ramifié ou un groupement alkoxy (C₁-C₆) linéaire ou ramifié pour conduire aux composés de formule (V) : 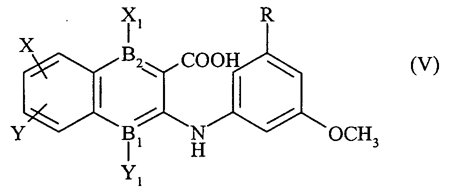 dans laquelle X, Y, X₁, Y₁, B₁, B₂ et R sont tels que définis dans la formule (I),
composés de formule (V) qui sont mis en présence d'acide polyphosphorique pour conduire aux composés de formule (VI) : 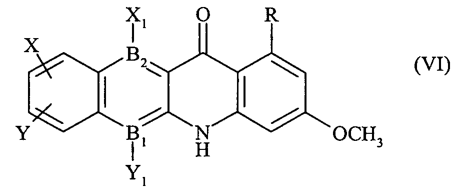 dans laquelle X, Y, X₁, Y₁, B₁, B₂ et R sont tels que définis précédemment,
***composés de formule (VI)*** **:**
**a)** dont l'atome d'azote est substitué, par action d'un halogénure d'alkyle ou d'un sulfate de dialkyle en présence d'un agent de déprotonation dans un solvant polaire aprotique pour conduire aux composés de formule (VII) : 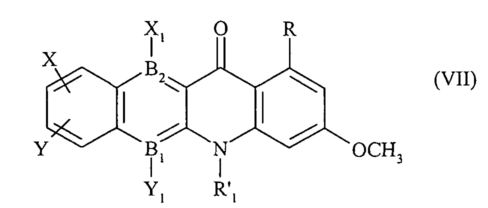 dans laquelle R'₁ représente un groupement alkyle (C₁-C₆) linéaire ou ramifié et X, Y, X₁, Y₁, B₁, B₂ et R sont tels que définis précédemment,
composés de formule (VII) qui sont traités par une solution d'acide bromhydrique dans de l'acide acétique, pour conduire aux composés de formule (VIII) : 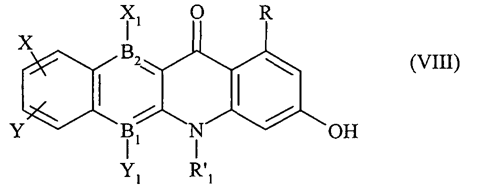 dans laquelle X, Y, X₁, Y₁, B₁, B₂, R et R'₁ sont tels que définis précédemment,
composés de formule (VIII) qui sont ensuite traités en condition basique dans un solvant aprotique anhydre par un alcyne de formule (IX) : 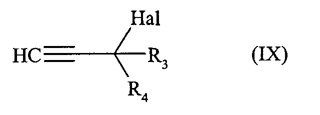 dans laquelle Hal représente un atome d'halogène et R₃ et R₄ sont tels que définis dans la formule (I), pour conduire aux composés de formule (X) : 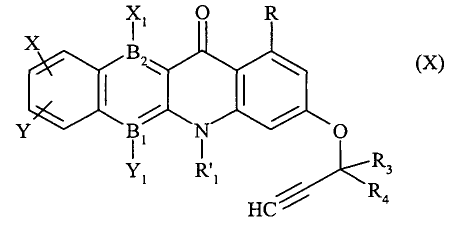 dans laquelle X, Y, X₁, Y₁, B₁, B₂, R, R'₁, R₃ et R₄ sont tels que définis précédemment,
composés de formule (X) qui sont mis sous reflux dans du diméthylformamide anhydre pour conduire aux composés de formule (I/a), cas particulier des composés de formule (I) : 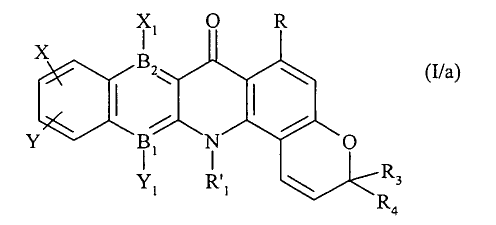 dans laquelle X, Y, X₁, Y₁, B₁, B₂, R, R'₁, R₃ et R₄ sont tels que définis précédemment,
**b)** soit soumis aux mêmes conditions que les composés de formule (VII) pour conduire aux composés de formule (XI) : 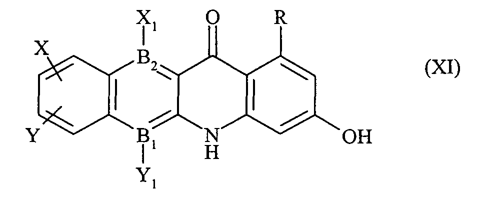
dans laquelle X, Y, X₁, Y₁, B₁, B₂ et R sont tels que définis précédemment,
composés de formule (XI) qui sont soumis successivement aux mêmes conditions que les composés de formule (VIII) et (X) pour conduire aux composés de formule (I/b), cas particulier des composés de formule (I) : 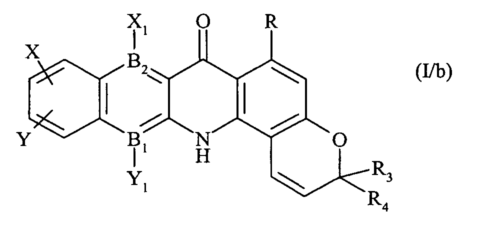 dans laquelle X, Y, X₁, Y₁, B₁, B₂, R, R₃ et R₄ sont tels que définis précédemment,
l'ensemble des composés (I/a) et (I/b) formant les composés de formule (I/c), cas particulier des composés de formule (I) : 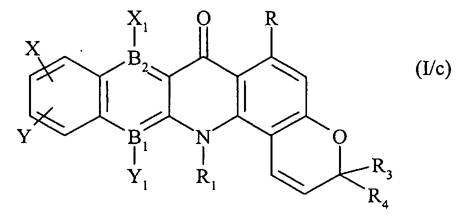 dans laquelle R₁ est tel que défini dans la formule (I) et X, Y, X₁, Y₁, B₁, B₂, R, R₃ et R₄ sont tels que définis précédemment,
composés de formule (I/c) qui sont éventuellement soumis à l'action d'un agent alkylant ou d'un agent acylant pour conduire aux composés de formule (I/d), cas particulier des composés de formule (I) : 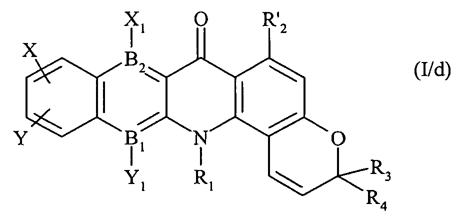 dans laquelle X, Y, X₁, Y₁, B₁, B₂, R₁, R₃ et R₄ sont tels que définis précédemment, et R'₂ représente un groupement choisi parmi -OR"ₐ₁, O-Tₐ-OR"ₐ, -O-C(O)-TₐH, -O-Tₐ-NR'ₐR'_{b} dans lesquels R'ₐ, R'_{b}, R"ₐ et Tₐ sont tels que définis dans la formule (I) et R"ₐ₁ représente un groupement alkyle (C₁-C₆) linéaire ou ramifié,
composés de formule (I/d) qui sont éventuellement traités, dans le cas où R'₂ représente un groupement
-OR"ₐ₁ dans lequel R"ₐ₁ est tel que défini précédemment par un composé de formule (XII) :
HNR₁₀R₁₁ (XII)
dans laquelle R₁₀ représente un groupement choisi parmi R'ₐ et R"ₐ et R₁₁ représente un groupement choisi parmi R'_{b}, -Tₐ-NR'ₐR'_{b}, -C(O)-TₐH, -Tₐ-OR"ₐ, -Ta-CO₂R"ₐ et -C(O)-Tₐ-NR'ₐR'_{b}, dans lesquels R'ₐ, R'_{b}, R"ₐ et Tₐ sont tels que définis précédemment, pour conduire aux composés de formule (I/e), cas particulier des composés de formule (I) : 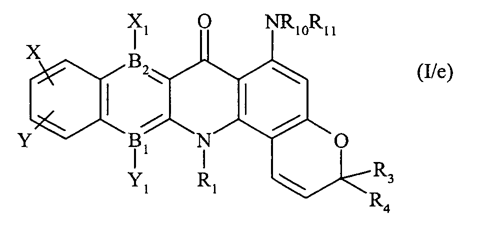 dans laquelle X, Y, X₁, Y₁, B₁, B₂, R₁, R₃ R₄, R₁₀ et R₁₁ sont tels que définis précédemment,
l'ensemble des composés de formule (I/c), (I/d) et (I/e) formant les composés de formule (I/f), cas particulier des composés de formule (I) : 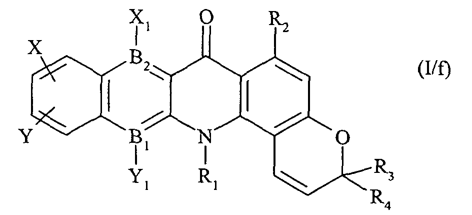 dans laquelle X, Y, X₁, Y₁, B₁, B₂, R₁, R₂, R₃ et R₄ sont tels que définis dans la formule (I),
***composés de formule*** **(*****I*****/*****f*****)** ***pouvant être soumis,***
**a)** soit à l'action d'un agent réducteur, pour conduire aux composés de formule (I/g), cas particulier des composés de formule (I) : 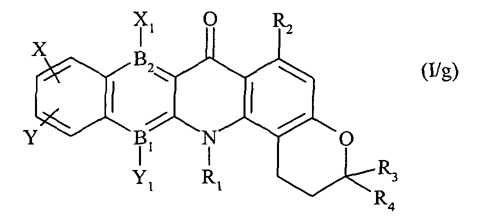 dans laquelle X, Y, X₁, Y₁, B₁, B₂, R₁, R₂, R₃ et R₄ sont tels que définis précédemment,
**b)** soit à l'action du tétroxyde d'osmium en milieu polaire et en présence de 4-méthylmorpholine-N-oxyde, pour conduire aux composés de formules (I/h₁) et (I/h₂), cas particulier des composés de formule (I) : 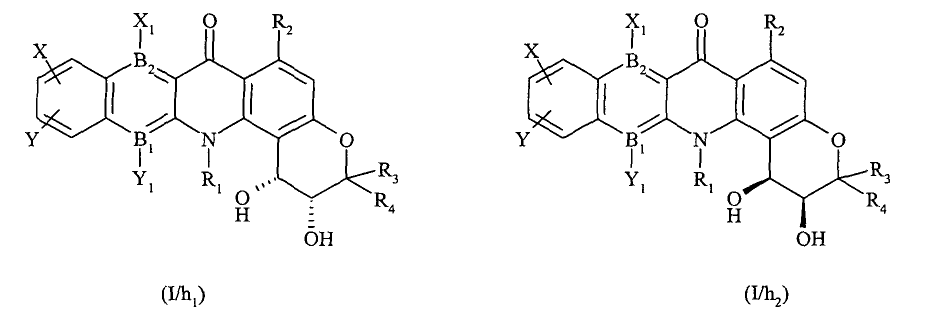 dans laquelle X, Y, X₁, Y₁, B₁, B₂, R₁, R₂, R₃ et R₄ sont tels que définis précédemment, l'ensemble des composés de formule (I/h₁) et (I/h₂) formant les composés cis-diol de formule (*cis*-I/h)*,* cas particulier des composés de formule (I) : 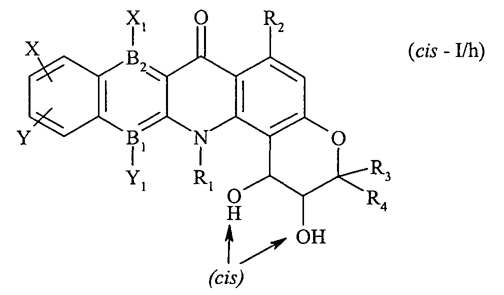 dans laquelle X, Y, X₁, Y₁, B₁, B₂, R₁, R₂, R₃ et R₄ sont tels que définis précédemment,
composés *cis*-diol de formule (*cis*-I/h) qui sont soumis éventuellement à l'action d'un composé de formule (XIII) : 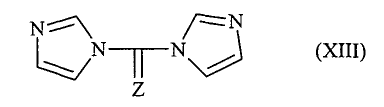 dans laquelle Z est tel que défini dans la formule (I) pour conduire aux composés de formule (*cis*-I/i), cas particulier des composés de formule (I) : 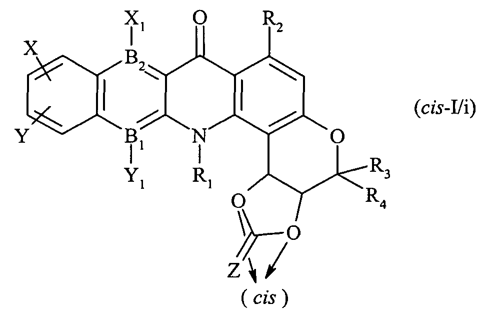 dans laquelle X, Y, X₁, Y₁, B₁, B₂, R₁, R₂, R₃ et R₄ sont tels que définis précédemment,
**c)** soit à l'action du permanganate de potassium en milieu polaire, pour conduire aux composés de formule (I/j), cas particulier des composés de formule (I) : 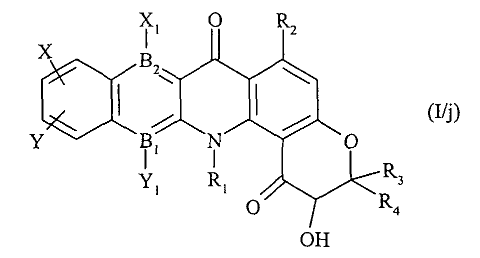 dans laquelle X, Y, X₁, Y₁, B₁, B₂, R₁, R₂, R₃ et R₄ sont tels que définis précédemment,
**composés de formule (I/j) pouvant être soumis** **:**
α) soit à l'action d'un agent alkylant ou d'un agent acylant, pour conduire aux composés de formule (I/k), cas particulier des composés de formule (I) : 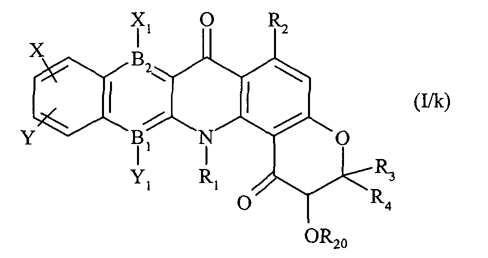 dans laquelle X, Y, X₁, Y₁, B₁, B₂, R₁, R₂, R₃ et R₄ sont tels que définis précédemment, et R₂₀ représente un groupement choisi parmi alkyle (C₁-C₆) linéaire ou ramifié ou alkylcarbonyl (C₁-C₆) linéaire ou ramifié,
β) soit à l'action d'un groupement protecteur P_{G} pour la fonction hydroxy puis à l'action de P₂S₅, pour conduire aux composés de formule (XIV) : 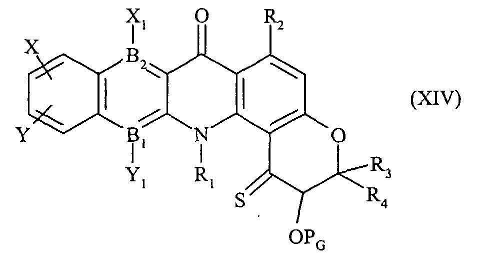 dans laquelle X, Y, X₁, Y₁, B₁, B₂, R₁, R₂, R₃, R₄ et P_{G} sont tels que définis précédemment, composés de formule (XIV) qui sont traités par un agent de réduction, puis soumis à une réaction de déprotection de la fonction hydroxy, pour conduire aux composés de formule (I/1), cas particulier des composés de formule (I) : 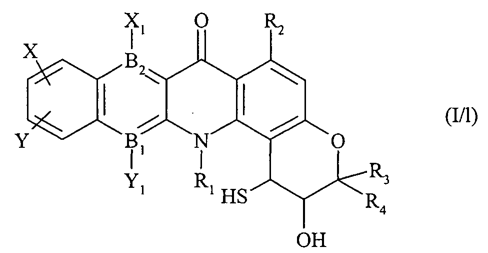 dans laquelle X, Y, X₁, Y₁, B₁, B₂, R₁, R₂, R₃ et R₄ sont tels que définis précédemment,
composés de formule (I/1) qui sont éventuellement soumis à l'action d'un composé de formule (XV) :
Hal - G₁ (XV)
dans laquelle Hal représente un atome d'halogène et G₁ représente un groupement choisi parmi -R_{c}, -C(W₂)-U-V, -C(W₂)-W₃-T₁, -S(O)ₙ-W₃-T₁ et -S(O)ₙ-T₁ dans lesquels R_{c}, W₂, W₃, U, V, T₁ et n sont tels que définis dans la formule (I), pour conduire aux composés de formule (I/m), cas particulier des composés de formule (I) : 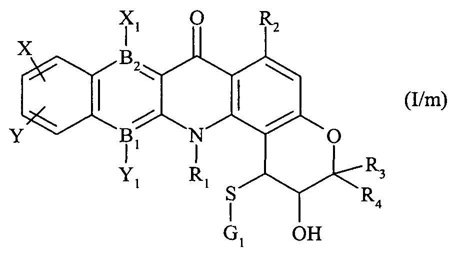 dans laquelle X, Y, X₁, Y₁, B₁, B₂, R₁, R₂, R₃, R₄, et G₁ sont tels que définis précédemment,
composés de formule (I/m) qui peuvent être soumis à l'action d'un composé de formule (XVI) :
Hal-G'₁ (XVI)
dans laquelle Hal est tel que défini précédemment et G'₁ représente un groupement choisi parmi -R_{c}, -C(W₂)-U-V, -C(W₂)-W₃-T₁, -S(O)ₙ-W₃-T₁ et -S(O)ₙ-T₁ dans lesquels R_{c}, W₂, W₃, U, V, T₁ et n sont tels que définis dans la formule (I), pour conduire aux composés de formule (I/n), cas particulier des composés de formule (I) : 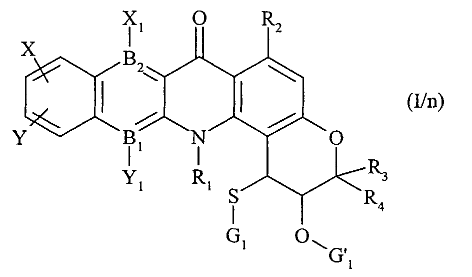 dans laquelle X, Y, X₁, Y₁, B₁, B₂, R₁, R₂, R₃, R₄, G₁ et G'₁ sont tels que définis précédemment,
γ) soit à des conditions réductrices en présence de NaBH₄, pour conduire aux composés de formule (I/o), cas particulier des composés de formule (I) : 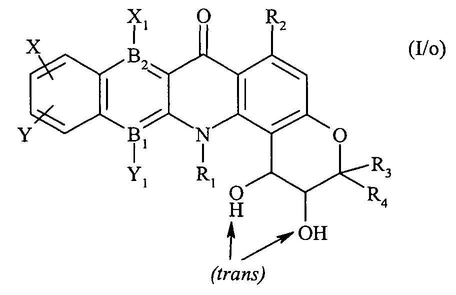
dans laquelle X, Y, X₁, Y₁, B₁, B₂, R₁, R₂, R₃, et R₄ sont tels que définis précédemment,
l'ensemble des composés de formules (*cis*-I/h) et (I/o) formant les composés de formule (I/p), 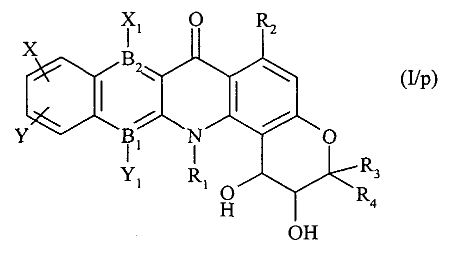 dans laquelle X, Y, X₁, Y₁, B₁, B₂, R₁, R₂, R₃, et R₄ sont tels que définis précédemment,
**composés de formule (I/p) pouvant être soumis** **:**
α) soit à l'action d'un composé de formule (XVI) tel que défini précédemment, pour conduire aux composés de formule (I/q₁), (I/q₂) et (I/q₃), cas particuliers des composés de formule (I) : 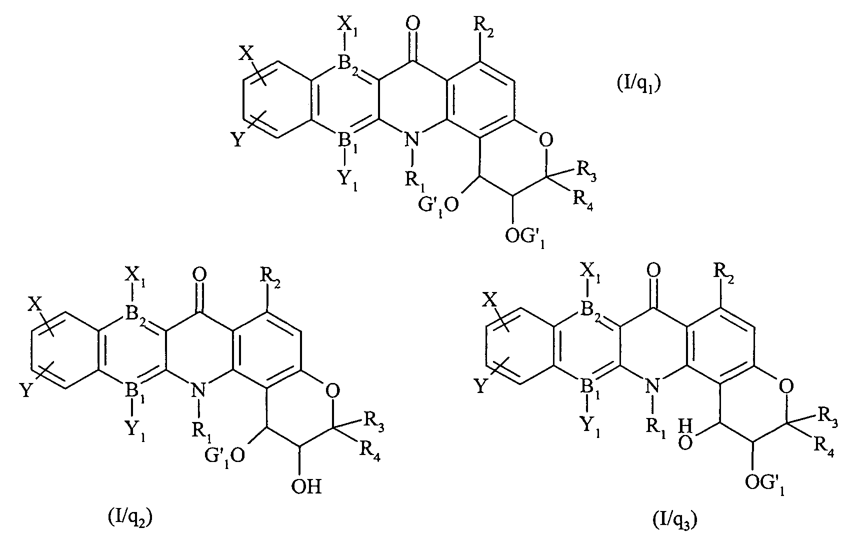 dans lesquelles X, Y, X₁, Y₁, B₁, B₂, R₁, R₂, R₃, R₄ et G'₁ sont tels que définis précédemment,
*composés de formules (I*/*q2) et* (*I*/*q*_{*3*}) *qui sont éventuellement soumis* *:*
***1)*** soit à l'action d'un alcool de formule R₃₀-OH dans laquelle R₃₀ représente un groupement alkyle (C₁-C₆) linéaire ou ramifié, pour conduire respectivement aux composés de formule (I/r₂) et (I/r₃), cas particuliers des composés de formule (I) : 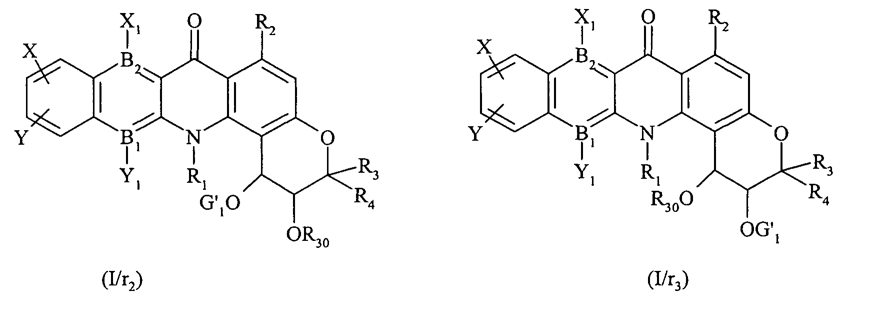 dans lesquelles X, Y, X₁, Y₁, B₁, B₂, R₁, R₂, R₃, R₄, G'₁ et R₃₀ sont tels que définis précédemment,
***2)*** soit à l'action d'un anhydride de formule (XVII) :
[V-U-C(W₂)]₂O (XVII)
dans laquelle W₂, U et V sont tels que définis dans la formule (I), pour conduire aux composés de formules (I/s₂) et (I/s₃), cas particuliers des composés de formule (I) : 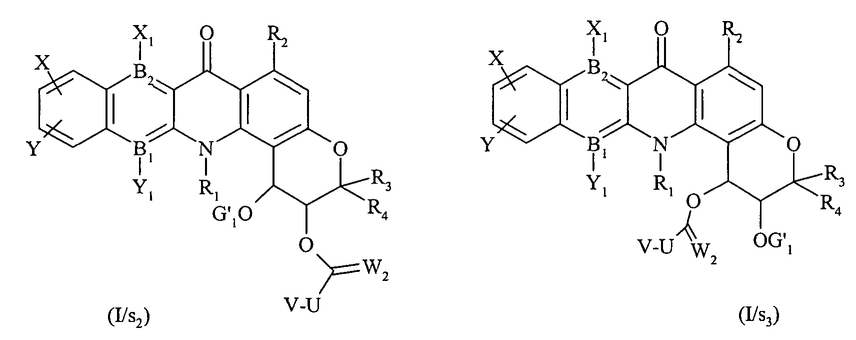 dans lesquelles X, Y, X₁, Y₁, B₁, B₂, R₁, R₂, R₃, R₄, G'₁, W₂, U et V sont tels que définis précédemment,
***3)*** soit à des conditions de déshydratation en milieu acide, pour conduire aux composés de formule (I/t₂) et (I/t₃), cas particuliers des composés de formule (I) : 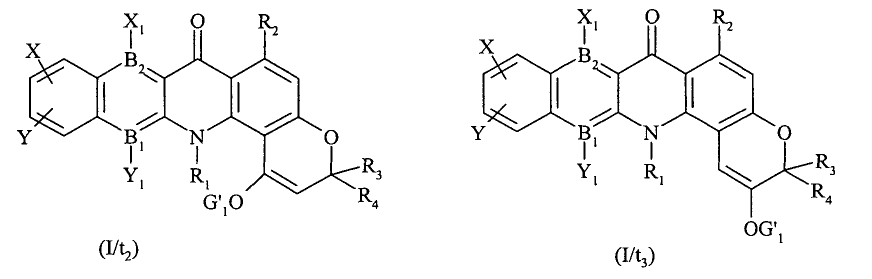 dans lesquelles X, Y, X₁, Y₁, B₁, B₂, R₁, R₂, R₃, R₄ et G'₁ sont tels que définis précédemment,
β) soit à l'action d'un composé de formules (XVIII) ou (XIX) 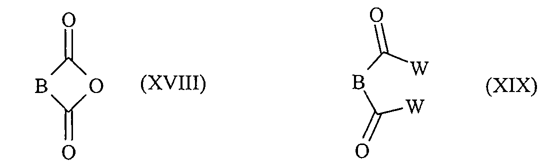 dans laquelle B est tel que défini dans la formule (I) et W représente un atome d'halogène ou un groupement hydroxyle, pour conduire aux composés de formule (I/u), cas particulier des composés de formule (I) : 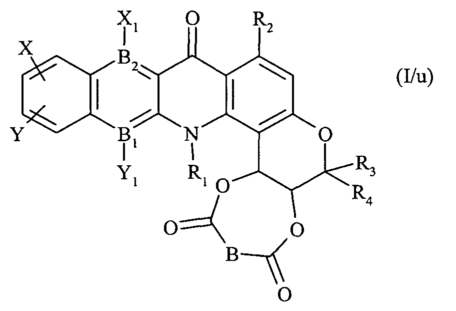 dans laquelle X, Y, X₁, Y₁, B₁, B₂, R₁, R₂, R₃, R₄ et B sont tels que définis précédemment,
γ) soit à l'action d'un dihalogénure d'alkyle (C₁-C₆) linéaire, pour conduire aux composés de formule (I/v), cas particulier des composés de formule (I) : 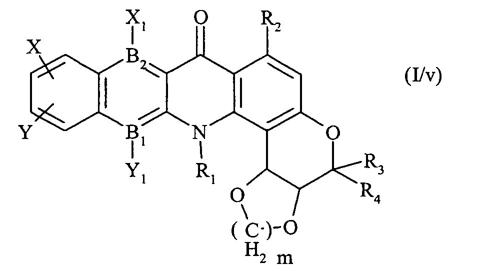 dans laquelle X, Y, X₁, Y₁, B₁, B₂, R₁, R₂, R₃ et R₄ sont tels que définis précédemment, et m est tel que défini dans la formule (I),
ε) soit à des conditions de déshydratation en milieu acide, pour conduire aux composés de formule (I/w), cas particulier des composés de formule (I) : 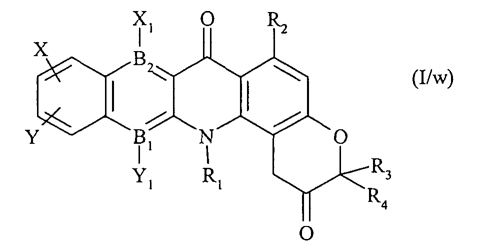 dans laquelle X, Y, X₁, Y₁, B₁, B₂, R₁, R₂, R₃ et R₄ sont tels que définis précédemment,
composés de formule (I/w) qui sont éventuellement réduits en présence de NaBH₄, pour conduire aux composés de formule (I/x), cas particulier des composés de formule (I) : 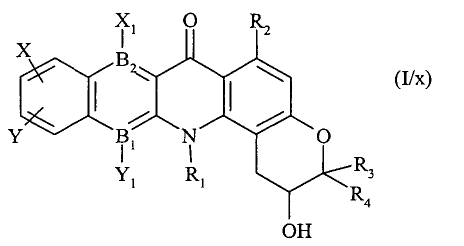 dans laquelle X, Y, X₁, Y₁, B₁, B₂, R₁, R₂, R₃ et R₄ sont tels que définis précédemment,
**d)** soit à l'action d'un péracide ou du diméthyle dioxirane, pour conduire aux composés de formule (I/y), cas particulier des composés de formule (I) : 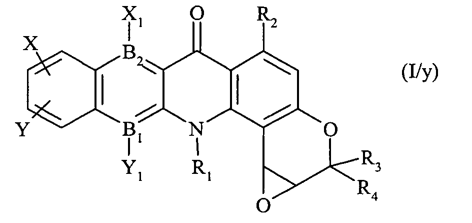 dans laquelle X, Y, X₁, Y₁, B₁, B₂, R₁, R₂, R₃ et R₄ sont tels que définis précédemment,
composés de formule (I/y) qui sont éventuellement traités par de l'ammoniac ou une amine primaire ou secondaire pour conduire aux composés de formules (I/z₁) et (I/z₂), cas particuliers des composés de formule (I) : 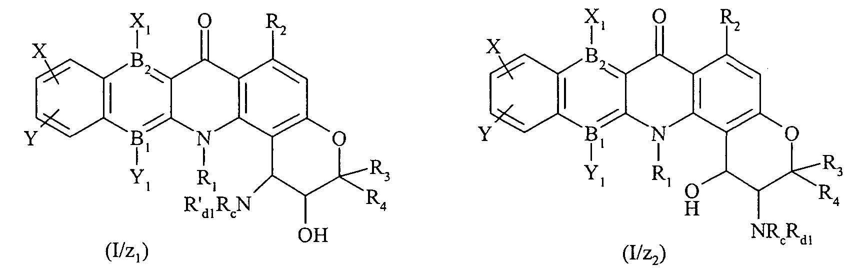 dans laquelle X, Y, X₁, Y₁, B₁, B₂, R₁, R₂, R₃ et R₄ sont tels que définis précédemment, R_{c} est tel que défini dans la formule (I) et R_{d1} représente un groupement choisi parmi R_{d}, -C(W₂)-U-V, -C(W₂)-W₃-T₁, -S(O)ₙ-W₃-T₁ et -S(O)ₙ-T₁ dans lesquels R_{d}, W₂, W₃, U, V et T₁ sont tels que définis précédemment,
**composés de formules (I/z**_{**1**}**) et (I/z**_{**2**}**) pouvant être soumis**
α) soit, dans le cas où R_{c} et R_{d1} représentent un atome d'hydrogène, à l'action d'un composé de formule (XIII) tel que défini précédemment, pour conduire aux composés de formules (I/aa₁) et (I/aa₂), cas particuliers des composés de formule (I) : 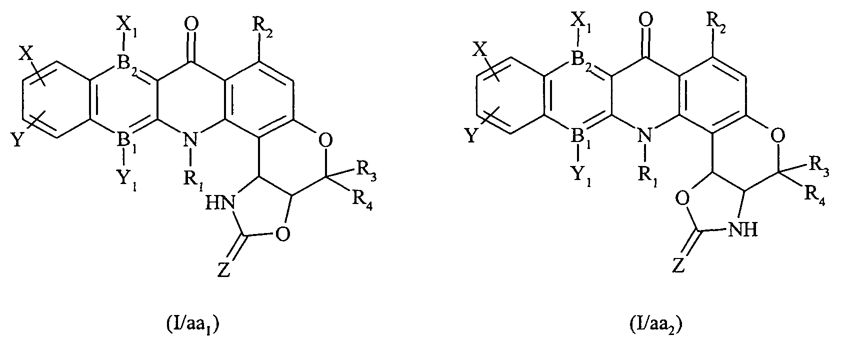 dans lesquelles X, Y, X₁, Y₁, B₁, B₂, R₁, R₂, R₃, R₄ et Z sont tels que définis précédemment,
β) soit à l'action d'un composé de formule (XIX) tel que défini précédemment, pour conduire aux composés de formules (I/ab₁) et (I/ab₂), cas particuliers des composés de formule (I) : 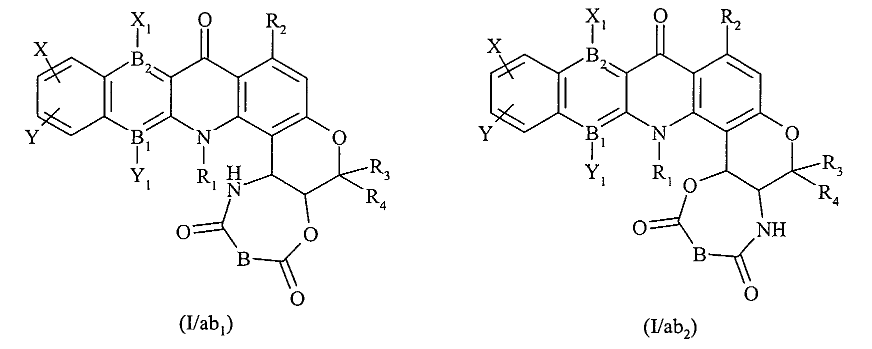 dans lesquelles X, Y, X₁, Y₁, B₁, B₂, R₁, R₂, R₃, R₄ et B sont tels que définis précédemment,
γ) soit à l'action d'un composé de formule (XVI) tel que défini précédemment, pour conduire aux composés de formules (I/ac₁) et (I/ac₂), cas particuliers des composés de formule (I) : 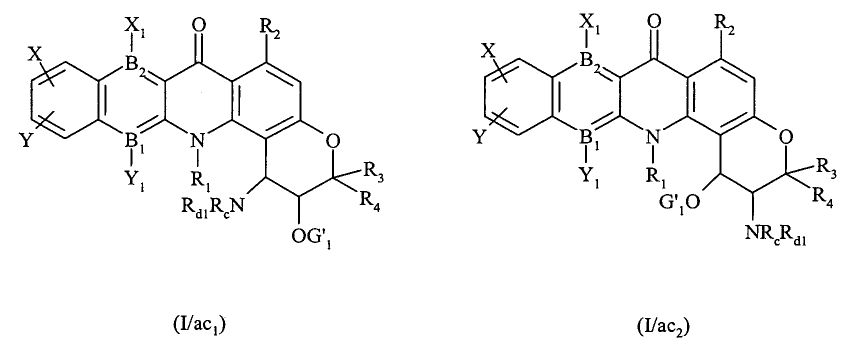 dans lesquelles X, Y, X₁, Y₁, B₁, B₂, R₁, R₂, R₃, R₄, R_{c}, R_{d1} et G'₁ sont tels que définis précédemment,
δ) soit à l'action de dibromure de triphénylphosphine en présence de triéthylamine pour conduire aux composés de formules (I/ad), cas particulier des composés de formule (I) : 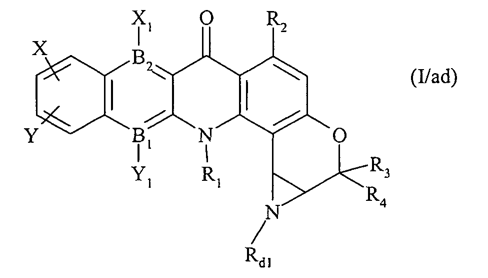 dans laquelle X, Y, X₁, Y₁, B₁, B₂, R₁, R₂, R₃, R₄ et R_{d1} sont tels que définis précédemment,
ε) soit à l'action de NaN₃ en présence d'eau oxygénée, suivi d'une étape de réduction, pour conduire aux composés de formules (I/ae), cas particulier des composés de formule (I) : 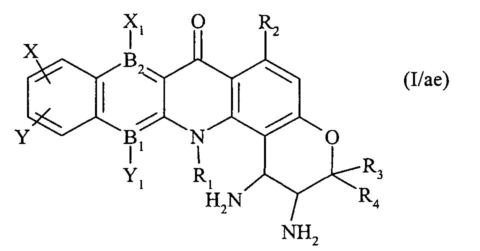
dans laquelle X, Y, X₁, Y₁, B₁, B₂, R₁, R₂, R₃ et R₄ sont tels que définis précédemment,
*composés de formule (Ilae) qui sont éventuellement soumis*
***1)*** soit à l'action du dioxyde de carbone, en présence de diphényl phosphite, pour conduire aux composés de formule (I/af), cas particulier des composés de formule (I) : 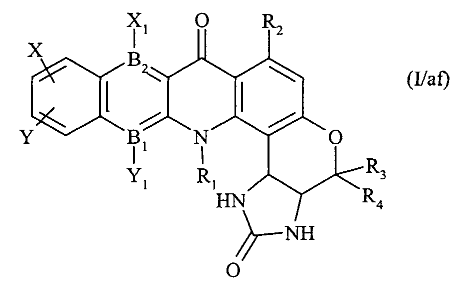 dans laquelle X, Y, X₁, Y₁, B₁, B₂, R₁, R₂, R₃ et R₄ sont tels que définis précédemment,
***2)*** soit à l'action d'un composé de formule (XVI) tel que défini précédemment, pour conduire aux composés de formules (Vag₁), (I/ag₂) et (I/ag₃), cas particulier des composés de formule (I) : 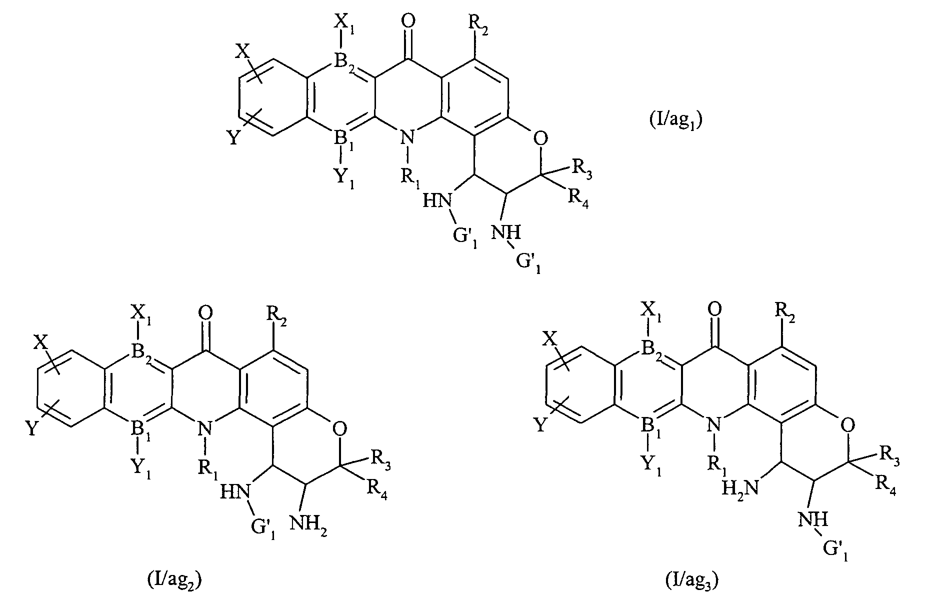 dans laquelle X, Y, X₁, Y₁, B₁, B₂, R₁, R₂, R₃, R₄ et G'₁ sont tels que définis précédemment,
composés de formules (I/ag₂) et (I/ag₃) dont la fonction amine primaire est protégée par un groupement protecteur des groupes amines primaires pour conduire aux composés de formules (XX/a) et (XX/b), 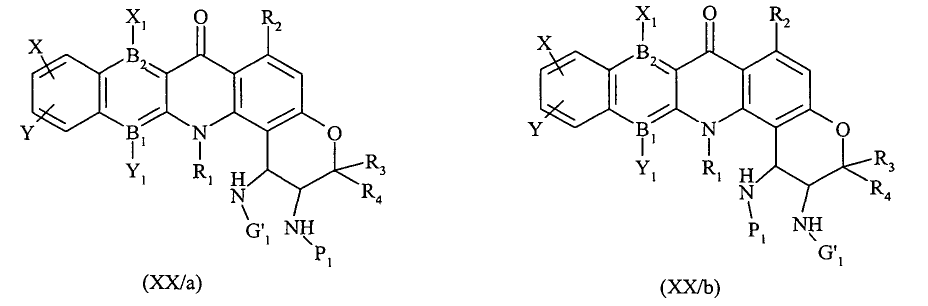 dans laquelle X, Y, X₁, Y₁, B₁, B₂, R₁, R₂, R₃ R₄, G'₁ sont tels que définis précédemment et P₁ représente un groupement protecteur de fonctions amines primaires,
composés de formules (I/ag₁) et (XX/a) et (XX/b) qui sont soumis à l'action d'un composé de formule (XXI) :
R_{c1}-Hal (XXI)
dans laquelle Hal représente un halogène et R_{c1} représente un groupement choisi parmi alkyle (C₁-C₆) linéaire ou ramifié, aryle ou arylalkyle (C₁-C₆) linéaire ou ramifié, puis qui sont soumis, dans le cas de composés de formule (XX/a) et (XX/b), à des conditions de déprotection de la fonction amine primaire, pour conduire aux composés de formules (I/ah₁), (I/ah₂) et (I/ah₃), cas particuliers des composés de formule (I) : 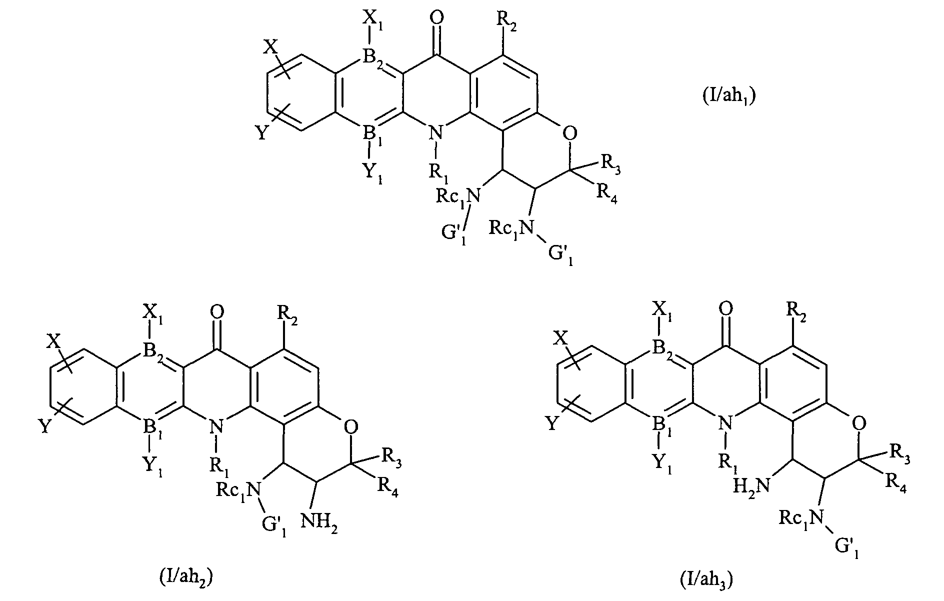 dans laquelle X, Y, X₁, Y₁, B₁, B₂, R₁, R₂, R₃ R₄, G'₁ et R_{c1} sont tels que définis précédemment,
composés de formules (I/ah₂) et (I/ah₃) qui sont éventuellement soumis successivement à l'action d'un composé de formule (XXI) tel que défini précédemment puis d'un composé de formule (XXII) :
R_{d'1}-Hal (XXII)
dans laquelle Hal est tel que défini précédemment et R_{d'1} peut prendre les même définitions que R_{c1}, pour conduire aux composés de formules (I/ai₂) et (I/ai₃), cas particuliers des composés de formule (I) : 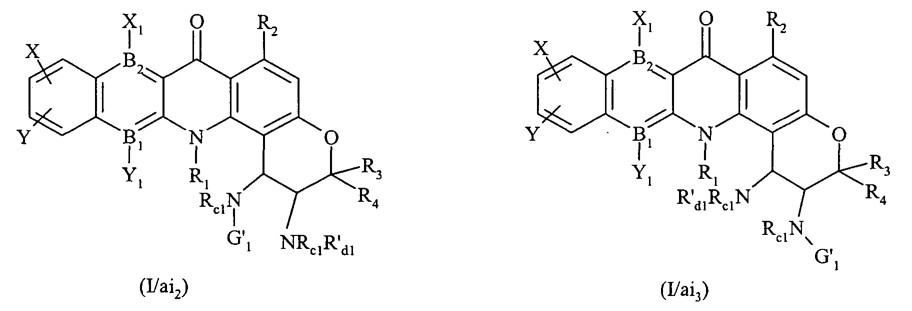 dans laquelle X, Y, X₁, Y₁, B₁, B₂, R₁, R₂, R₃, R₄, G'₁, R_{c1} et R_{d1} sont tels que définis précédemment,
φ) soit à des conditions de déshydratation en milieu acide, pour conduire aux composés de formules (I/aj₂) et (I/aj₃), cas particuliers des composés de formule (I) : 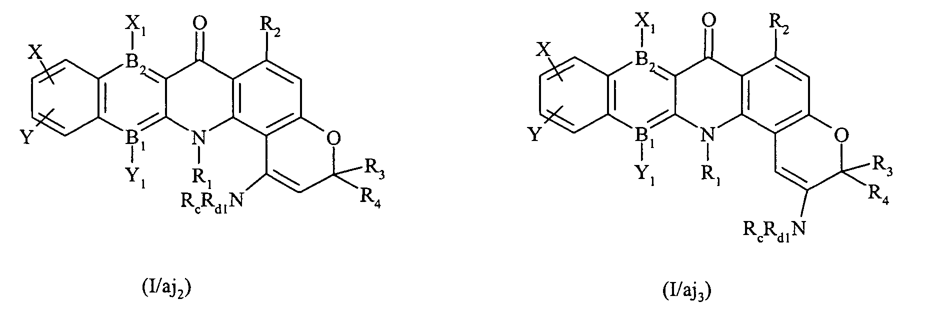 dans laquelle X, Y, X₁, Y₁, B₁, B₂, R₁, R₂, R₃, R₄, R_{c} et R_{d1} sont tels que définis précédemment,
**e)** soit à l'action d'un composé de formule (XXIII) :
Hal-C(W₂)-T₁ (XXIII)
dans laquelle Hal représente un atome d'halogène et W₂ et T₁ sont tels que définis dans la formule (I), pour conduire aux composés de formule (I/aj), cas particulier des composés de formule (I) : 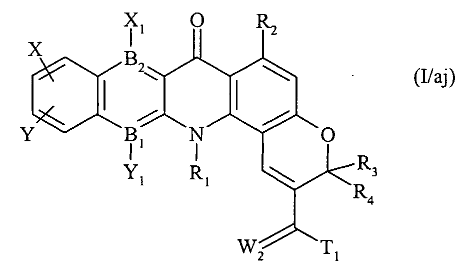 dans laquelle X, Y, X₁, Y₁, B₁, B₂, R₁, R₂, R₃ R₄, W₂ et T₁ sont tels que définis précédemment,
les composés (I/a) à (I/aj) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

21. Compositions pharmaceutiques contenant comme principe actif au moins un composé selon l'une quelconque des revendications 1 à 19, en combinaison avec un ou plusieurs excipients où véhicules inertes, non toxiques, pharmaceutiquement acceptables.

22. Compositions pharmaceutiques selon la revendication 21 contenant au moins un principe actif selon l'une quelconque des revendication 1 à 19 utiles en tant que médicaments, dans le traitement des cancers.
